# EUROPÄISCHE PATENTSCHRIFT

(11) **EP 0 973 921 B1**
(45) Veröffentlichungstag und Bekanntmachung des Hinweises auf die Patenterteilung: **05.09.2001**
(21) Anmeldenummer: 98919245.5
(22) Anmeldetag: 09.04.1998
(51) Int. Cl.: C12N 15/82, C12N 9/16

(54) **2-DESOXYGLUCOSE-6-PHOSPHAT (2-DOG-6-P) PHOSPHATASE DNA-SEQUENZEN ALS SELEKTIONSMARKER IN PFLANZEN**
2-DEOXYGLUCOSE-6-PHOSPHATE (2-DOG-6-P) PHOSPHATASE DNA SEQUENCES AS SELECTION MARKERS IN PLANTS
SEQUENCES D'ADN CODANT POUR UNE 2-DESOXYGLUCOSE-6-PHOSPHATE (2-DOG-6-P) PHOSPHATASE ET SERVANT DE MARQUEUR DE SELECTION CHEZ LES VEGETAUX

(30) Priorität: 09.04.1997 EP 97105855
(43) Veröffentlichungstag der Anmeldung: 26.01.2000
(73) Patentinhaber: IPK Gatersleben, 06466 Gatersleben (DE)
(72) Erfinder: SONNEWALD, Uwe, D-06484 Quedlinburg (DE); EBNETH, Marcus, D-12163 Berlin (DE)
(74) Vertreter: VOSSIUS & PARTNER
(86) Internationale Anmeldenummer: EP9802069
(87) Internationale Veröffentlichungsnummer: WO9845456

(56) Entgegenhaltungen:
- EP-A- 0 289 478
- WO-A-94/20627
- WO-A-96/31612
- F. RANDEZ-GIL ET AL: "DOG(R)1 and DOG(R)2: two genes from Saccharomyces cerevisiae that confer 2-deoxyglucose resistance when overexpressed" YEAST, Bd. 11, Nr. 13, Oktober 1995, NEW YORK US, Seiten 1233-1240, XP002040749
- DATABASE WPI Week 7709 Derwent Publications Ltd., London, GB; AN 77-15528Y XP002040752 & JP 52 007 423 (MEIJI SEIKA CO) , 2.Januar 1977 & JP 52 007 423 A
- G STENLID: "Species differences between plant roots in the reaction to inhibitory sugars" PHYSIOLOGICA PLANTARUM, Bd. 12, 1959, COPENHAGEN DK, Seiten 218-235, XP002040750 in der Anmeldung erwähnt
- J ZEMEK ET AL.: "Effect of 2-deoxy-d-glucose on tissue culture of Nicotiana tabacum L. (cv. Virginia Bright Italia)" ZEITSCHRIFT FÜR PLANZENPHYSIOLOGIE, Bd. 76, Nr. 2, 1975, STUTTGART DE, Seiten 114-119, XP002040751 in der Anmeldung erwähnt

### SEQUENZPROTOKOLL

(1) ALLGEMEINE ANGABEN:
   (i) ANMELDER:
      (A) NAME: IPK Gatersleben
      (B) STRASSE: Corrensstr. 3
      (C) ORT: Gatersleben
      (E) LAND: Germany
      (F) POSTLEITZAHL: 06466
   (ii) BEZEICHNUNG DER ERFINDUNG: 2-DOG-6-P Phosphatase DNA-Sequenzen als Selektionsmarker in Pflanzen
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 758 Basenpaare
      (B) ART: Nucleotid
      (C) STRANGFORM: Einzelstrang
      (D) TOPOLOGIE: linear
   (ii) ART DES MOLEKÜLS: cDNA
   (iii) HYPOTHETISCH: NEIN
   (iv) ANTISENSE: NEIN
   (vi) URSPRÜNGLICHE HERKUNFT:
      (A) ORGANISMUS: Saccharomyces cerevisiae
   (ix) MERKMAL:
      (A) NAME/SCHLÜSSEL: CDS
      (B) LAGE:9..746
   (xi) SEQUENZBESCHREIBUNG: SEQ ID NO: 1:
   GGATCCCC ATG GCA GAA TTT TCA GCT GAT CTA TGT CTT TTT GAC CTA GAT        50
            Met Ala Glu Phe Ser Ala Asp Leu Cys Leu Phe Asp Leu Asp
              1               5                  10
   GGT ACC ATA GTG AGT ACA ACA GTG GCC GCA GAG AAA GCA TGG ACC AAG          98
   Gly Thr Ile Val Ser Thr Thr Val Ala Ala Glu Lys Ala Trp Thr Lys
    15                  20                  25                  30
   TTG TGT TAC GAA TAC GGT GTT GAT CCT TCC GAG TTA TTT AAG CAT TCT         146
   Leu Cys Tyr Glu Tyr Gly Val Asp Pro Ser Glu Leu Phe Lys His Ser
                   35                  40                  45
   CAT GGT GCA AGA ACA CAA GAG GTT TTG AGA AGG TTT TTC CCT AAA TTG      194
   His Gly Ala Arg Thr Gln Glu Val Leu Arg Arg Phe Phe Pro Lys Leu
               50                  55                  60
   GAT GAT ACA GAC AAT AAA GGT GTT CTT GCT CTA GAA AAA GAT ATT GCC      242
   Asp Asp Thr Asp Asn Lys Gly Val Leu Ala Leu Glu Lys Asp Ile Ala
           65                  70                  75
   CAT AGT TAC TTG GAT ACA GTA AGC CTT ATT CCT GGT GCA GAG AAC TTA      290
   His Ser Tyr Leu Asp Thr Val Ser Leu Ile Pro Gly Ala Glu Asn Leu
       80                  85                  90
   CTG TTA TCG TTA GAT GTA GAT ACT GAG ACT CAA AAA AAG TTA CCT GAA      338
   Leu Leu Ser Leu Asp Val Asp Thr Glu Thr Gln Lys Lys Leu Pro Glu
   95                  100                 105                 110
   AGG AAA TGG GCT ATC GTT ACC TCT GGT TCT CCA TAT TTG GCA TTT TCA      386
   Arg Lys Trp Ala Ile Val Thr Ser Gly Ser Pro Tyr Leu Ala Phe Ser
                   115                 120                 125
   TGG TTC GAG ACA ATA TTG AAA AAT GTT GGA AAG CCC AAA GTT TTC ATT      434
   Trp Phe Glu Thr Ile Leu Lys Asn Val Gly Lys Pro Lys Val Phe Ile
               130                 135                 140
   ACT GGG TTT GAC GTG AAG AAC GGT AAG CCT GAT CCC GAG GGT TAT TCA      482
   Thr Gly Phe Asp Val Lys Asn Gly Lys Pro Asp Pro Glu Gly Tyr Ser
           145                 150                 155
   AGA GCT CGT GAT TTA TTG CGT CAA GAT TTG CAA TTA ACT GGT AAA CAG      530
   Arg Ala Arg Asp Leu Leu Arg Gln Asp Leu Gln Leu Thr Gly Lys Gln
       160                 165                 170
   GAT CTG AAG TAT GTT GTC TTC GAA GAT GCA CCC GTG GGC ATA AAG GCC      578
   Asp Leu Lys Tyr Val Val Phe Glu Asp Ala Pro Val Gly Ile Lys Ala
   175                 180                 185                 190
   GGC AAA GCA ATG GGC GCC ATT ACT GTG GGT ATA ACA TCC TCG TAT GAC      626
   Gly Lys Ala Met Gly Ala Ile Thr Val Gly Ile Thr Ser Ser Tyr Asp
                   195                 200                 205
   AAG AGC GTT TTA TTT GAC GCA GGA GCA GAT TAT GTA GTC TGT GAT TTG      674
   Lys Ser Val Leu Phe Asp Ala Gly Ala Asp Tyr Val Val Cys Asp Leu
               210                 215                 220
   ACA CAG GTT TCC GTG GTT AAG AAC AAT GAA AAC GGT ATT GTC ATC CAG      722
   Thr Gln Val Ser Val Val Lys Asn Asn Glu Asn Gly Ile Val Ile Gln
           225                 230                 235
   GTA AAC AAC CCT TTG ACA AGG GCC TGAGTAGTCG AC                        758
   Val Asn Asn Pro Leu Thr Arg Ala
       240                 245
(2) ANGABEN ZU SEQ ID NO: 2:
   (i) SEQUENZKENNZEICHEN:
      (A) LÄNGE: 246 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ala Glu Phe Ser Ala Asp Leu Cys Leu Phe Asp Leu Asp Gly Thr
    1               5                  10                  15
   Ile Val Ser Thr Thr Val Ala Ala Glu Lys Ala Trp Thr Lys Leu Cys
               20                  25                  30
   Tyr Glu Tyr Gly Val Asp Pro Ser Glu Leu Phe Lys His Ser His Gly
           35                  40                  45
   Ala Arg Thr Gln Glu Val Leu Arg Arg Phe Phe Pro Lys Leu Asp Asp
       50                  55                  60
   Thr Asp Asn Lys Gly Val Leu Ala Leu Glu Lys Asp Ile Ala His Ser
   65                  70                  75                  80
   Tyr Leu Asp Thr Val Ser Leu Ile Pro Gly Ala Glu Asn Leu Leu Leu
                   85                  90                  95
   Ser Leu Asp Val Asp Thr Glu Thr Gln Lys Lys Leu Pro Glu Arg Lys
               100                 105                 110
   Trp Ala Ile Val Thr Ser Gly Ser Pro Tyr Leu Ala Phe Ser Trp Phe
           115                 120                 125
   Glu Thr Ile Leu Lys Asn Val Gly Lys Pro Lys Val Phe Ile Thr Gly
       130                 135                 140
   Phe Asp Val Lys Asn Gly Lys Pro Asp Pro Glu Gly Tyr Ser Arg Ala
   145                 150                 155                 160
   Arg Asp Leu Leu Arg Gln Asp Leu Gln Leu Thr Gly Lys Gln Asp Leu
                   165                 170                 175
   Lys Tyr Val Val Phe Glu Asp Ala Pro Val Gly Ile Lys Ala Gly Lys
               180                 185                 190
   Ala Met Gly Ala Ile Thr Val Gly Ile Thr Ser Ser Tyr Asp Lys Ser
           195                 200                 205
   Val Leu Phe Asp Ala Gly Ala Asp Tyr Val Val Cys Asp Leu Thr Gln
       210                 215                 220
   Val Ser Val Val Lys Asn Asn Glu Asn Gly Ile Val Ile Gln Val Asn
   225                 230                 235                 240
   Asn Pro Leu Thr Arg Ala
                   245
(2) ANGABEN ZU SEQ ID NO: 3:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (iii) HYPOTHETISCH: JA
   (iv) ANTISENSE: NEIN
   (xi) SEQUENZBESCHREIBUNG: SEQ ID NO: 3:
   ATGGATCCCC ATGGCAGAAT TTTCAGCTGA TCTATG                    36
(2) ANGABEN ZU SEQ ID NO: 4:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (iii) HYPOTHETISCH: JA
   (iv) ANTISENSE: NEIN
   (xi) SEQUENZBESCHREIBUNG: SEQ ID NO: 4:
   ATGTCGACTA CTCAGGCCCT TGTCAAAGGG TTG                       33

## Beschreibung

Die vorliegende Erfindung betrifft die Verwendung von DNA-Sequenzen, die ein Protein mit der biologischen Aktivität einer 2-Desoxyglucose-6-Phosphat (2-DOG-6-P) Phosphatase codieren, als Selektionsmarker in Pflanzenzellen zur Selektion transformierter Pflanzen. Weiterhin betrifft diese Erfindung rekombinante DNA-Moleküle, die derartige DNA-Sequenzen enthalten, wobei diese mit regulatorischen Sequenzen eines in Pflanzen aktiven Promotors und Transkriptions-Terminations- und/oder Polyadenylierungssignalen operativ verbunden sind. Ferner werden Vektoren, Wirtszellen und Kits beschrieben, die derartige rekombinante DNA-Moleküle enthalten, sowie mit den rekombinanten DNA-Molekülen transformierte Pflanzenzellen und Pflanzen. Die vorliegende Erfindung betrifft ebenfalls Verfahren zur Herstellung transgener Pflanzen, die aufgrund der Einführung der oben beschriebenen rekombinanten DNA-Moleküle auf 2-Desoxyglucose enthaltenden Medien selektioniert werden können. Schließlich betrifft die vorliegende Erfindung transgene Pflanzen, Pflanzenzellen und Gewebe, die das erfindungsgemäße DNA-Molekül enthalten oder durch das vorstehend beschriebene Verfahren erhalten werden, sowie Ernteprodukte und Vermehrungsmaterial der beschriebenen transgenen Pflanzen.

Mittels gentechnischer Verfahren ist es möglich, gezielt Fremdgene in das pflanzliche Genom zu übertragen. Dieser Prozeß wird als Transformation und die resultierenden Pflanzen als transgen bezeichnet. Die vornehmlichen Ziele sind zum einen der Pflanzenschutz und zum anderen eine Qualitätssteigerung der Ernteprodukte. Beispiele für Pflanzenschutzmaßnahmen sind: (i) herbizidtolerante Pflanzen (DE-A-3701623; Stalker (1988) Science 242, 419), (ii) insektenresistente Pflanzen (Vaek (1987) Plant Cell 5, 159-169), (iii) virusresistente Pflanzen (Powell (1986) Science 232, 738-743) und (vi) ozon-resistente Pflanzen (Van Camp (1994) BioTech. 12, 165-168). Beispiele für Qualitätssteigerungen sind: (i) Erhöhung der Haltbarkeit von Früchten (Oeller (1991) Science 254, 437-439), (ii) Erhöhung der Stärkeproduktion in Kartoffelknollen (Stark (1992) Science 242, 419), (iii) Veränderung der Stärke- (Visser (1991) Mol. Gen. Genet. 225, 289-296) und Lipidzusammensetzung (Voelker (1992) Science 257, 72-74) und (iv) Produktion Pflanzenfremder Polymere (Poirer (1992) Science 256, 520-523).

Grundvoraussetzung für die Erzeugung transgener Pflanzen ist die Verfügbarkeit geeigneter Transformationssysteme und das Vorhandensein selektionierbarer Marker, die die Identifizierung erfolgreich transformierter Pflanzenzellen ermöglichen.
Zur Transformation stehen derzeit mehrere Verfahren zur Verfügung. Die am häufigsten eingesetzte Methode zur Tranformation dikotyledoner Pflanzen ist der Agrobacterium-vermittelte Gentransfer. Hierbei wird die natürliche Fähigkeit des Bodenbakteriums ausgenutzt, genetisches Material in das pflanzliche Genom zu integrieren. Weitere geeignete Verfahren sind beispielsweise ProtoplastenTransformation durch Polyethylenglykol-induzierte DNA-Aufnahme, Elektroporation, Sonikation oder Mikroinjektion sowie die Transformation intakter Zellen oder Gewebe durch Mikro- oder Makroinjektion in Gewebe oder Embryonen, Gewebeelektroporation, Inkubation trockener Embryonen in DNA-haltiger Lösung, Vakuuminfiltration von Samen und biolistischer Gentransfer.
Da unabhängig vom Transformationsverfahren nur wenige Zellen die gewünschten Eigenschaften tragen, wird in herkömmlicher Weise neben dem Zielgen ein selektionierbarer Marker in das pflanzliche Genom integriert, der die Identifizierung transgener Zellen ermöglicht. Derzeit werden zur Selektion transformierter Pflanzenzellen vornehmlich Gene eingesetzt, die eine Herbizid- oder Antibiotikatoleranz vermitteln. Geeignete Resistenzgene sind beispielsweise das bar-Gen aus Streptomyces hygroscopicus, das Resistenz gegen das Totalherbizid Phosphinothricin vermittelt (De Block (1987) EMBO J. 6, 2513-2518) oder das nptll-Gen aus dem Transposon Tn5 von Escherichia coli, das Resistenz gegen das Antibiotikum Kanamycin bewirkt (Herrera-Estrella (1983) EMBO J. 2, 987-995). Je nach Pflanzenart, sind die genannten Verfahren nicht immer effektiv und beeinflussen häufig die Pflanzenregeneration negativ. Darüber hinaus ist der Einsatz von Genen, die Antibiotikaresistenzen vermitteln, im Lebensmittelbereich unerwünscht. Desweiteren ist zur Steuerung komplexer Stoffwechselprozesse die Manipulation mehrerer enzymatischer Schritte notwendig. D.h. die Möglichkeit transgene Pflanzen mehrfach zu transformieren ist im Bereich des "Metabolic Engineering" essentiell. Die genannten Gründe haben dazu geführt, daß die Suche nach weiteren selektionierbaren Markern verstärkt vorangetrieben wird. Trotz intensiver Bemühungen sind nur wenige neue Marker zur Selektion transformierter Pflanzenzellen erfolgreich eingesetzt worden. Durch Expression einer Mannose-6-Phosphat Isomerase konnte eine positive Selektion auf Mannose-haltigen Nährmedien für transformierte Pflanzenzellen etabliert werden (WO 94/20627). Ein weiteres Verfahren nutzt die Fähigkeit einer Deaminase aus Aspergillus terreus aus, das Insektizid Blasticidin S zu detoxifizieren (Tamura (1995) Biosci. Biotechnol. Biochem. 59, 2336-2338).

Der vorliegenden Erfindung liegt somit die Aufgabe zugrunde, rekombinante DNA-Moleküle zur Verfügung zu stellen, die eine DNA-Sequenz enthalten, die zur Selektion transformierter Pflanzenzellen oder Pflanzen eingesetzt werden kann.
Diese Aufgabe wird durch die Bereitstellung der in den Patentansprüchen gekennzeichneten Ausführungsformen gelöst.

Somit betrifft die vorliegende Erfindung ein rekombinantes DNA-Molekül umfassend
(a) regulatorische Sequenzen eines in Pflanzen aktiven Promotors;
(b) operativ daran gebunden eine DNA-Sequenz, die ein Protein mit der biologischen Aktivität einer 2-Desoxyglucose-6-Phosphat (2-DOG-6-P) Phosphatase codiert; und
(c) operativ daran gebunden regulatorische Sequenzen, die als Transkriptions-Terminations- und/oder Polyadenylierungssignale in Pflanzen dienen können.

Im Zusammenhang mit der vorliegenden Erfindung wird unter einem Protein mit der biologischen Aktivität einer 2-DOG-6-P Phosphatase ein Protein verstanden, das in der Lage ist, nicht-metabolisierbare glucoseanaloge Verbindungen wie 2-DOG in nicht-toxische Produkte umzusetzen. Die toxische Wirkung von 2-DOG entsteht nach Phosphorylierung zum 2-DOG-6-P, d.h. die Phosphatase hebt die Wirkung von 2-DOG-6-P durch Dephosphorylierung auf. Ein alternativer Resistenzmechanismus, der ebenfalls im Sinne der vorliegenden Erfindung genutzt werden kann, besteht darin, die Phosphorylierung oder die Aufnahme von 2-DOG zu unterbinden. Entsprechende Mutanten wurden in Hefe beschrieben, z.B. eine Transportmutante (Novak (1990) FEBS Lett. 269, 202-204) und eine Phosphorylierungsmutante (Lobo (1977) Mol. Gen. Genet. 157, 297-300).

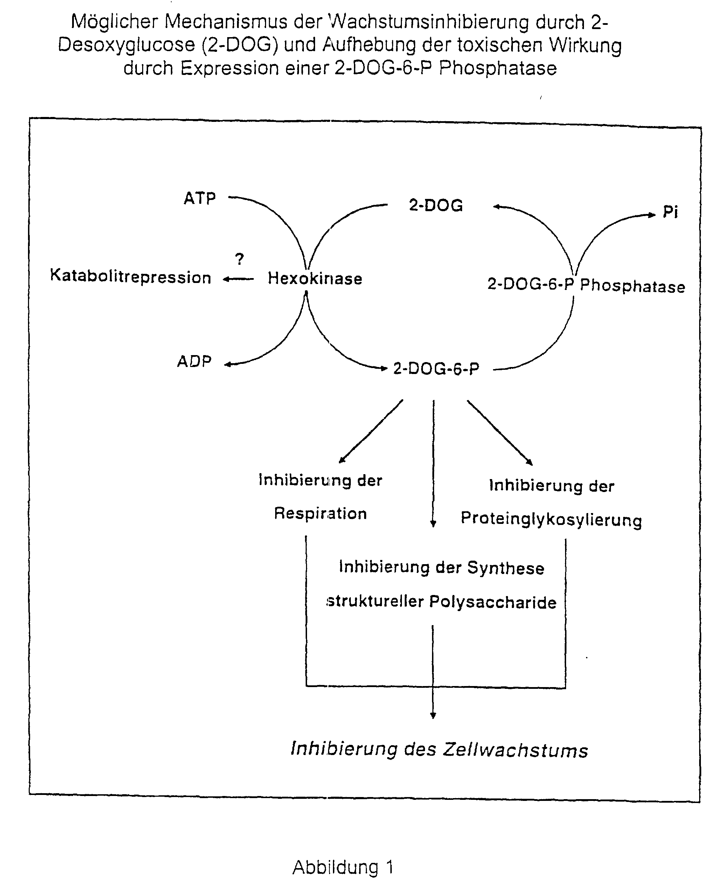

Überraschenderweise wurde gefunden, daß die Expression einer 2-DOG-6-P Phosphatase aus Hefe Resistenz gegenüber 2-DOG vermitteln und zur Selektion transformierter Pflanzen eingesetzt werden kann, die ansonsten phänotypisch normal und fertil sind. Aus Untersuchungen in Hefe war bekannt, daß Zugabe von 2-DOG, einem nicht-metabolisierbaren Glucoseanalogon, zur Inhibierung der Respiration und des Wachstums von Zellen führt (Heredia (1964) Biochem. Biophys.
Acta 86, 216). In Hefezellen korreliert die Wachstumsinhibierung mit einer verminderten Synthese struktureller Polysaccharide (Kratky (1975) Eur. J. Biochem. 54, 459) und einer Blockierung der Proteinglykosylierung (Datema & Schwarz (1978) Eur. J. Biochem. 90, 505), die vermutlich durch Veränderungen der Zuckernucleotidkonzentrationen hervorgerufen werden. Über die biochemischen Änderungen hinaus führt die Zugabe von 2-DOG zu einer Reprimierung einer Vielzahl von Genen (zusammengefaßt in Gancedo (1992) Eur. J. Biochem. 206, 297). Analog zur Wirkungsweise metabolisierbarer Zucker (wie z.B. Glucose), wird dieser Prozeß als Katabolitrepression bezeichnet.
Untersuchungen an Hefezellen haben gezeigt, daß der 2-DOG Wirkmechanismus von der intrazellulären 2-DOG-6-P Konzentration abhängig ist. Molekulare Charakterisierung von Hefemutanten, die resistent gegenüber 2-DOG sind, ergab, daß die erworbene Resistenz auf der Überexpression einer spezifischen 2-DOG-6-P Phosphatase beruht (Sanz (1994) Yeast 10, 1195). Die hypothetische Wirkungsweise sowie der Wirkmechanismus der 2-DOG-6-P Phosphatase ist in Abbildung 1 dargestellt.
Im Gegensatz zu den zahlreichen Arbeiten über die Wirkungsweise von 2-DOG in tierischen Geweben, in Hefen und Bakterien gibt es allerdings nur wenige analoge Studien, die sich mit dem Stoffwechsel dieser Zucker in Pflanzen befassen. Bisher konnte in einigen Untersuchungen lediglich gezeigt werden, daß Zugabe von 2-DOG das Wurzelwachstum einer Vielzahl von Pflanzen hemmt (u.a. Flachs, Wicke, Klee, Roggen, Gerste, Mais und Hafer (Stenlid (1959) Pnysioi. Plant. 12, 218; Farrar (1995) J. Exp. Bot. 46, 1859)). Darüber hinaus wird das Wachstum von Nicotiana tabacum-Zellen und Picea excelsa (Fichte)-Zellen in Gewebekultur durch 2-DOG stark gehemmt (Zemek (1975) Z. Pflanzenphysiol. 76, 114; Zemek (1976) Z. Pflanzenphysiol. 77, 95). Als Umsetzungsprodukte von 2-DOG in höheren Pflanzen konnte 2-DOG-1-P, 2-DOG-6-P, UDP-2-DOG sowie 2-DOG enthaltende Di- und Oligosaccharide nachgewiesen werden (Kocourek (1963) Biochem. Biophys. Acta 71, 497; Zemek (1975) Z. Pflanzenphysiol. 76, 114). Die Bildung dieser Stoffwechselprodukte wird für die inhibitorische Wirkung der 2-DOG verantwortlich gemacht. Für andere toxische Verbindungen, die als Selektionsmarker in der Pflanzentransformation eingesetzt werden, sind ebenfalls vielfältige Wirkungen auf den Metabolismus der Pflanze beschrieben. So führt z. B. die Zugabe von manchen Antibiotika oftmals zur Regeneration von transgenen Pflanzen, die physiologische und morphologische Veränderungen gegenüber dem Phänotyp des Wildtyps aufweisen und/oder steril sind.
Obwohl wie vorstehend erläutert auch 2-DOG eine Vielzahl von teilweise noch unbekannten biochemischen Änderungen in Zellen hervorruft, werden bei der Verwendung der erfindungsgemäßen rekombinanten DNA-Moleküle in Verbindung mit der Selektion auf 2-DOG enthaltenden Medien phänotypisch normale und fertile transgene Pflanzen regeneriert.

Die DNA-Sequenz, die ein Protein mit der biologischen Aktivität einer 2-DOG-6-P Phosphatase codiert, kann aus natürlichen Quellen isoliert werden, vorzugsweise Hefe, oder kann nach bekannten Verfahren synthetisiert werden.
Mittels gängiger molekularbiologischer Techniken ist es möglich (siehe z.B. Sambrook, 1989, Molecular Cloning, A Laboratory Manual, 2. Aufl. Cold Spring Harbor Laboratory Press, Cold Spring Harbor, NY), verschiedenartige Mutationen in die DNA-Sequenz, die ein Protein mit der biologischen Aktivität einer 2-DOG-6-P Phosphatase codiert, in den erfindungsgemäßen rekombinanten DNA-Molekülen einzuführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletionen vom 5'- oder vom 3'- Ende der codierenden DNA-Sequenz die Synthese entsprechend verkürzter Proteine erreicht werden kann. Ferner ist es möglich, gezielt Enzyme herzustellen, die durch Addition entsprechender Signalsequenzen in bestimmten Kompartimenten der Pflanzenzelle lokalisiert sind. Derartige Sequenzen sind bekannt (siehe beispielsweise Braun, EMBO J. 11 (1992), 3219-3227; Wolter, Proc. Natl. Acad. Sci. USA 85 (1988), 846-850; Sonnewald, Plant J. 1 (1991), 95-106).
Andererseits ist auch die Einführung von Punktmutationen denkbar an Positionen, bei denen eine Veränderung der Aminosäuresequenz einen Einfluß beispielsweise auf die Enzymaktivität oder die Regulierung des Enzyms hat. Auf diese Weise können z.B. Mutanten hergestellt werden, die einen veränderten Kₘ-Wert besitzen oder nicht mehr den normalerweise in der Zelle vorliegenden Regulationsmechanismen über allosterische Regulation oder kovalente Modifizierung unterliegen.
Des weiteren können Mutanten hergestellt werden, die eine veränderte Substrat- oder Produktspezifität aufweisen. Weiterhin können Mutanten hergestellt werden, die ein verändertes Aktivitäts-Temperatur-Profil aufweisen.
Für die gentechnische Manipulation in prokaryontischen Zellen können die erfindungsgemäßen rekombinanten DNA-Moleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook, 1989, Molecular Cloning: A Laboratory Manual, 2. Aufl., Cold Spring Harbor Laboratory Press, NY, USA) können Basenaustausche vorgenommen oder natürliche oder synthetische Sequenzen hinzugefügt werden. Für die Verbindung der DNA-Fragmente untereinander können an die Fragmente Adaptoren oder Linker angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können in vitro-Mutagenese, "primer repair", Restriktion oder Ligation verwendet werden. Als Analysemethode werden im allgemeinen eine Sequenzanalyse, eine Restriktionsanalyse und weitere biochemisch-molekularbiologische Methoden durchgeführt.

In einer bevorzugten Ausführungsform ist die DNA-Sequenz, die ein Protein mit der biologischen Aktivität einer 2-DOG-6-P Phosphatase codiert, ausgewählt aus der Gruppe bestehend aus
(a) DNA-Sequenzen, die eine Nucleotid-Sequenz umfassen, die die in SEQ ID NO. 2 angegebene Aminosäuresequenz codieren;
(b) DNA-Sequenzen, die die in SEQ ID NO. 1 angegebene Nucleotidsequenz umfassen;
(c) DNA-Sequenzen umfassend eine Nucleotidsequenz, die mit einem komplementären Strang der Nucleotidsequenz von (a) oder (b) hybridisieren;
(d) DNA-Sequenzen umfassend eine Nucleotidsequenz, die zu einer Nucleotidsequenz von (c) degeneriert ist, und
(e) DNA-Sequenzen, die ein Derivat, Analog oder Fragment einer Nucleotidsequenz von (a), (b), (c) oder (d) ist und für ein Protein codiert, das 2-DOG-6-P Phosphatase Aktivität besitzt.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook (Molecular Cloning, A Laboratory Manual, 2. Aufl. (1989) Cold Spring Harbor Laboratory Press, Cold Spring Harbor, NY) beschrieben sind.
DNA-Sequenzen, die mit den DNA-Sequenzen, die ein Protein mit der biologischen Aktivität einer 2-DOG-6-P Phosphatase codieren, hybridisieren, können z.B. aus genomischen oder aus cDNA-Bibliotheken isoliert werden, die aus Hefe hergestellt wurden. Die Identifizierung und Isolierung derartiger DNA-Sequenzen kann dabei, z.B. mittels Hybridisierung nach Standardverfahren (siehe z.B. Sambrook, 1989, Molecular Cloning, A Laboratory Manual, 2. Aufl. Cold Spring Harbor Laboratory Press, Cold Spring Harbor, NY), z.B. unter Verwendung der DNA-Sequenzen erfolgen, die exakt oder im wesentlichen die unter SEQ ID NO. 1 angegebene Nucleotidsequenz oder Teile dieser Sequenzen aufweisen bzw. die reversen Komplemente dieser DNA-Sequenzen aufweisen. Bei den als Hybridisierungsprobe verwendeten Fragmenten kann es sich auch um synthetische Fragmente handeln, die mit Hilfe üblicher Synthesetechniken hergestellt wurden und deren Sequenz im wesentlichen mit der in SEQ ID NO. 2 dargestellten übereinstimmt. Die DNA-Sequenzen, die ein Protein mit der biologischen Aktivität einer 2-DOG-6-P Phosphatase codieren, umfassen auch DNA-Sequenzen, deren Nucleotidsequenzen zu der einer der vorstehend beschriebenen DNA-Sequenzen degeneriert ist. Die Degeneration des genetischen Codes bietet dem Fachmann u.a. die Möglichkeit, die Nucleotidsequenz der DNA-Sequenz an die Codon-Präferenz des jeweiligen Wirts, vorzugsweise Pflanzen, anzupassen.
Die oben beschriebenen DNA-Sequenzen umfassen auch Fragmente, Derivate und allelische Varianten der oben beschriebenen DNA-Sequenzen, die ein Protein mit der biologischen Aktivität einer 2-DOG-6-P Phosphatase codieren. Unter "Fragmenten" werden dabei Teile der DNA-Sequenz verstanden, die lang genug sind, um eines der beschriebenen Proteine zu codieren. Der Ausdruck "Derivat" bedeutet in diesem Zusammenhang, daß die Sequenzen sich von den oben beschriebenen DNA-Sequenzen an einer oder mehreren Positionen unterscheiden aber einen hohen Grad an Homologie zu diesen Sequenzen aufweisen. Homologie bedeutet dabei eine Sequenzidentität von mindestens 40 %, insbesondere eine Identität von mindestens 60 %, vorzugsweise über 80 % und besonders bevorzugt über 90 %. Dabei weisen die durch diese DNA-Sequenzen codierten Proteine eine Sequenzidentität zu der in SEQ ID NO. 2 angegebenen Aminosäuresequenz von mindestens 80%, vorzugsweise 85% und besonders bevorzugt über 90%, 95%, 97% und 99% auf. Die Abweichungen zu den oben beschriebenen DNA-Sequenzen können dabei beispielsweise durch Deletion, Substitution, Insertion oder Rekombination entstanden sein.
Bei den DNA-Sequenzen, die homolog zu den oben beschriebenen Sequenzen sind und Derivate dieser Sequenzen darstellen, handelt es sich in der Regel um Variationen dieser Sequenzen, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.

Die von den verschiedenen Varianten der in den rekombinanten DNA-Molekülen enthaltenen DNA-Sequenzen codierten Proteine, die die biologische Aktivität einer 2-DOG-6-P Phosphatase besitzen, weisen bestimmte gemeinsame Charakteristika auf, wie Enzymaktivität, Molekulargewicht, immunologische Reaktivität oder Konformation oder physikalische Eigenschaften, wie das Laufverhalten in Gelelektrophoresen, chromatographisches Verhalten, Sedimentationskoeffizienten, Löslichkeit, spektroskopische Eigenschaften, Stabilität; pH-Optimum, Temperatur-Optimum.

In einer besonders bevorzugten Ausführungsform stammt die beschriebene DNA-Sequenz aus Hefe.

Zur Expression der in den erfindungsgemäßen rekombinanten DNA-Molekülen enthaltenen DNA-Sequenz in pflanzlichen Zellen ist diese mit regulatorischen Sequenzen verknüpft, die die Transkription in pflanzlichen Zellen gewährleisten. Hierzu zählen insbesondere Promotoren. Generell kommt für die Expression jeder in pflanzlichen Zellen aktive Promotor in Frage.
Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein. Sinnvolle Promotoren sind z.B. der Promotor der 35S RNA des Cauliflower Mosaic Virus und der Ubiquitin-Promotor aus Mais für eine konstitutive Expression, besonders bevorzugt ein Promotor, der eine Expression lediglich in photosynthetisch aktiven Geweben sicherstellt, z.B. der ST-LS1-Promotor (Stockhaus, Proc. Natl. Acad. Sci. USA 84 (1987), 7943-7947; Stockhaus, EMBO J. 8 (1989), 2445-2451) oder ein Promotor, der während der Pflanzentransformation, der Pflanzenregeneration oder bestimmten Stadien dieser Prozesse aktiv ist, z.B. zellteilungsspezifische Promotoren wie der Histon H-3 Promotor (Kapros (1993), In Vitro Cell Dev. Biol. Plant 29, 27-32) oder das chemisch-induzierbare Tet-System (Gatz (1991), Mol. Gen. Genet. 227, 229-237). Eine Übersicht weiterer in Frage kommender Promotoren ist z.B. in Ward (1993) Plant Mol. Biol. 22, 361-366 gegeben.

Weiterhin bevorzugt sind induzierbare oder zellspezifische Promotoren (z.B. Meristem).
Ferner ist eine Transkriptions-Terminationssequenz vorhanden, die der korrekten Beendigung der Transkription dient sowie der Addition eines Poly-A-Schwanzes an das Transkript dienen kann, dem eine Funktion bei der Stabilisierung der Transkripte beigemessen wird. Derartige Elemente, z.B. der Terminator des Octopinsynthasegens aus Agrobakterien, sind in der Literatur beschrieben (vgl. Gielen, EMBO J. 8 (1989), 23-29) und sind beliebig austauschbar.

In einer bevorzugten Ausführungsform ist der Promotor der 35S CAMV Promotor.

Die Erfindung betrifft ferner Vektoren, die erfindungsgemäße rekombinante DNA-Moleküle enthalten. Vorzugsweise handelt es sich dabei um Plasmide, Cosmide, Viren, Bacteriophagen und andere in der Gentechnik übliche Vektoren. Zur Vorbereitung der Einführung fremder Gene in höhere Pflanzen stehen eine große Anzahl von Clonierungsvektoren zur Verfügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird für die Transformation von E.coli-Zellen verwendet. Transformierte E.coli-Zellen werden in einem geeigneten Medium gezüchtet, anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden. Jede Plasmid-DNA-Sequenz kann in den gleichen oder anderen Plasmiden cloniert werden.

In einer bevorzugten Ausführungsform enthält der erfindungsgemäße Vektor mindestens ein weiteres rekombinantes DNA-Molekül. Durch die Bereitstellung der erfindungsgemäßen DNA-Moleküle und Vektoren kann jede beliebige Information, die in einem weiteren rekombinanten DNA-Molekül enthalten ist, auf Pflanzen bzw.

Pflanzenzellen übertragen werden und anschließend mitteis Seiektion auf 2-DOG enthaltenden Medien auf die gewünschte Information selektioniert werden. Natürlich weiß der Fachmann, daß das rekombinante DNA-Molekül, das die zusätzliche Information enthält, nicht unbedingt in dem Vektor, der den Selektionsmarker trägt, anwesend sein muß, sondern auch mit diesem co-transformiert werden kann (Lyznik (1989) Plant Mol. Biol. 13, 151-161; Peng (1995) Plant Mol. Biol. 27, 91-104). Diese Möglichkeit bietet sich zum Beispiel an, wenn keine physikalische Koppelung des Markergens und der zu übertragenden Information gewünscht wird. In diesem Fall können nach der Selektion der primären transgenen Pflanze das Markergen und die gewünschte Information in nachfolgenden Kreuzungen unabhängig voneinander seggregieren.

In einer besonders bevorzugten Ausführungsform enthält das weitere rekombinante DNA-Molekül eine DNA Sequenz, die ein Peptid, Protein, Antisense-, Sense-RNA, virale RNA, oder Ribozym codiert. Einige Beispiele zur heterologen (Über)expression und zur Antisense-Inhibierung mit dem Ziel, Stoffwechselflüsse in transgenen Pflanzen zu manipulieren, sind in Herbers & Sonnewald (1996) TIBTECH 14, 198-205 zusammengefaßt. Ein Beispiel für Ribozyme wurde von Feyter (1996) Mol. Gen. Genet. 250, 329-338 publiziert. Durch Expression eines "hammerhead" Ribozyms gelang es den Autoren, eine TMV-Resistenz in transgenen Tabakpflanzen zu erzeugen. Vielfältige Anwendungsmöglichkeiten von transgenen Pflanzen, die mit Hilfe der erfindungsgemäßen rekombinanten DNA-Moleküle und Vektoren erzeugt werden können, sind auch in TIPTEC Plant Product & Crop Biotechnology, 13 (1995), 312-397 beschrieben.
Die erfindungsgemäßen Vektoren können weitere Funktionseinheiten besitzen, die eine Stabilisierung des Vektors im Wirtsorganismus bewirken, wie einen bakteriellen Replikationsursprung oder die 2-Mikron-DNA zur Stabilisation in *Saccharomyces cerevisiae.* Ferner können "left border"- und "right border"-Sequenzen agrobakterieller T-DNA enthalten sein, wodurch eine stabile Integration in das Erbgut von Pflanzen ermöglicht wird. Eine weitere Strategie, Markerfreie transgene Pflanzen zu erzeugen, ist die Verwendung von Sequenz-spezifischen Rekombinasen. Zu diesem Zweck sind z.B. zwei Strategien einsetzbar: (i) Re-Transformation einer Rekombinase exprimierenden Ausgangslinie und Auskreuzung der Rekombinase nach erfolgter Entfernung des Selektionsmarkers, welches mit dem gewünschten Gen assoziiert war. (ii) Co-Transformation mit anschließender Auskreuzung. Voraussetzung für diese Rekombinase-Strategie sind (i) Flankierung des Selektionsmarkers mit Erkennungssequenzen für die Rekombinase und (ii) eine Rekombinase, die in Pflanzen aktiv ist und keine Pflanzen-eigenen Sequenzen zur Rekombination nutzt. Derartige Verfahren kann der Fachmann dem Stand der Technik entnehmen, z.B. RecA (Reiss (1996) Proc. Natl. Acad. Sci. USA 93, 3094-3098), Cre/lox (Bayley (1992) Plant Mol. Biol. 18, 353-361), FLP/FRT (Lloyd (1994) Mol. Gen. Genet. 242, 653-657), Gin (Maeser (1991) Mol. Gen. Genet. 230, 170-176) und R/RS (Onouchi (1991) Nucl. Acids Res. 19, 6373-6378).

In einer weiteren Ausführungsform betrifft die Erfindung Wirtszellen, die die erfindungsgemäßen rekombinanten DNA-Moleküle oder Vektoren transient oder stabil enthalten. Unter einer Wirtszelle wird ein Organismus verstanden, der in der Lage ist, *in vitro* rekombinierte DNA aufzunehmen und gegebenenfalls die in den erfindungsgemäßen rekombinanten DNA-Molekülen enthaltene DNA-Sequenz zu exprimieren.
Vorzugsweise sind dies prokaryontische oder eukaryontische Zellen. Insbesondere betrifft die Erfindung Pflanzenzellen, die die erfindungsgemäßen Vektorsysteme oder Derivate oder Teile davon enthalten. Diese sind vorzugsweise aufgrund der Aufnahme der erfindungsgemäßen Vektorsysteme, Derivate oder Teile der Vektorsysteme zu einer Synthese von Enzymen befähigt, die die biologische Aktivität einer 2-DOG-6-P Phosphatase besitzen. Vorzugsweise sind die erfindungsgemäßen Zellen dadurch gekennzeichnet, daß das eingeführte erfindungsgemäße rekombinante DNA-Molekül entweder heterolog in Bezug auf die transformierte Zelle ist, d.h. natürlicherweise nicht in diesen Zellen vorkommt, oder an einem anderen Ort im Genom lokalisiert ist als die entsprechende natürlicherweise auftretende Sequenz.

In einer weiteren Ausführungsform betrifft die Erfindung Kits, die ein erfindungsgemäßes rekombinantes DNA Molekül oder einen erfindungsgemäßen Vektor enthalten und gegebenenfalls 2-DOG oder eine zu 2-DOG äquivalente chemische Verbindung. So wurden z.B. von Schmidt (1978) (Eur. J. Biochem. 87, 55-68) 2-Desoxy-2-fluoro-D-glucose bzw. Mannose ais markierte Analoga beschrieben.

Durch die Bereitstellung der erfindungsgemäßen rekombinanten DNA-Moleküle und Vektoren ist es möglich, 2-DOG als Selektionsmittel für die Pflanzentransformation zu benutzen, vorzugsweise zur Selektion transformierter Pflanzen, die von Hochleistungssorten abstammen, für die die im Stand der Technik beschriebenen Selektionsmarker oftmals nur im beschränkten Maße von Nutzen sind.
Die vorliegende Erfindung betrifft somit auch ein Verfahren zur Selektion transformierter Pflanzenzellen, das die folgenden Schritte umfaßt:
(a) Gewinnung von Pflanzenzellen;
(b) Einführung eines erfindungsgernäßen rekombinanten DNA-Moleküls oder Vektors in diese Pflanzenzellen; und
(c) Selektion der erfolgreich transformierten Pflanzenzellen auf 2-DOG enthaltenden Medien oder auf Medien, die eine zu 2-DOG funktionell äquivalente chemische Verbindung enthalten.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, die Einbringung von DNA mittels der biolistischen Methode sowie weitere Möglichkeiten.
Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, sollte ein selektierbarer Marker vorhanden sein.
Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.

Werden für die Transformation Agrobakterien verwendet, muß die einzuführende DNA in spezielle Plasmide cloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Sefektionsmarker-Gen und einen Linker oder Polylinker, welche von der rechten und linken T-DNA Grenzregion eingerahmt werden. Sie können direkt in die Agrobakterien transformiert werden (Holsters, Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und beschrieben in EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46 und An, EMBO J. 4 (1985), 277-287.
In einer bevorzugten Ausführungsform wird der erfindungsgemäße Vektor in dem erfindungsgemäßen Verfahren mittels Agrobacterium tumefaciens in Pflanzenzellen übertragen.

Für den Transfer der DNA in die Pflanzenzelle können Pflanzen-Explantate zweckmäßigerweise mit Agrobacterium tumefaciens oder Agrobacterium rhizogenes kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches 2-DOG oder eine funktionell äquivalente Chemikalie zur Selektion transformierter Zellen enthält, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z.B. Christou (1996) Trends in Plant Science 1, 423-431; Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).

Alternative Systeme zur Transformation von monokotylen Pflanzen sind die Transformation mittels des biolistischen Ansatzes, die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimenden Pollen und die DNA-Aufnahme in Embryonen durch Quellung (zur Übersicht: Potrykus, Physiol. Plant (1990), 269 - 273).
Während die Transformation dikotyler Pflanzen über Ti-Plasmid-Vektorsysteme mit Hilfe von Agrobacterium tumefaciens wohl etabliert ist, weisen neuere Arbeiten darauf hin, daß auch monokotyle Pflanzen der Transformation mittels Agrobacterium basierender Vektoren sehr wohl zugänglich sind (Chan, Plant Mol. Biol. 22 (1993), 491-506; Hiei, Plant J. 6 (1994), 271-282; Bytebier, Proc. Natl. Acad. Sci. USA 84 (1987), 5345 - 5349; Raineri, Bio/Technology 8 (1990), 33 - 38; Gould, Plant Physiol. 95 (1991), 426 - 434; Mooney, Plant, Cell Tiss. & Org. Cult. 25 (1991), 209 - 218; Li, Plant Mol. Biol. 20 (1992), 1037 - 1048).
Drei der oben genannten Transformationssysteme konnten in der Vergangenheit für verschiedene Getreide etabliert werden: die Elektroporation von Gewebe, die Transformation von Protoplasten und der DNA-Transfer durch Partikel-Beschuß in regenerierbare Gewebe und Zellen (zur Übersicht: Jähne, Euphytica 85 (1995), 35-44). Die Transformation von Weizen wird in der Literatur verschiedentlich beschrieben (zur Übersicht: Maheshwari, Critical Reviews in Plant Science 14 (2) (1995), 149 - 178).

In einer anderen bevorzugten Ausführungsform wird das erfindungsgemäße rekombinante DNA-Molekül oder der erfindungsgemäße Vektor in dem erfindungsgemäßen Verfahren mittels Particle-Bombardment (biolistische Verfahren) übertragen.

Die vorliegende Erfindung betrifft auch transyene Fflanzenzellen, die ein erfindungsgemäßes rekombinantes DNA-Molekül oder einen erfindungsgemäßen Vektor enthalten oder durch das erfindungsgemäße Verfahren erhalten wurden sowie transgene Pflanzenzellen, die von derartig transformierten Pflanzenzellen abstammen. Derartige Zellen lassen sich von natürlicherweise vorkommenden Pflanzenzellen dadurch unterscheiden, daß sie mindestens ein erfindungsgemäßes rekombinantes DNA-Molekül enthalten, das natürlicherweise in diesen Zellen nicht vorkommt oder dadurch, daß ein solches Molekül an einem Ort im Genom der Zelle integriert vorliegt, in dem es natürlicherweise nicht vorkommt, d.h. in einer anderen genomischen Umgebung.

In einer bevorzugten Ausführungsform enthält die erfindungsgemäße Pflanzenzelle mindestens ein weiteres Fremdgen. Wie bereits in der Einleitung dieser Patentanmeldung erwähnt, ist für die Möglichkeit der Steuerung komplexer Stoffwechselprozesse die Manipulation mehrerer enzymatischer Schritte notwendig und daher die Möglichkeit transgene Pflanzen mehrfach zu transformieren essentiell. Mit Hilfe der erfindungsgemäßen rekombinanten DNA-Moleküle und Vektoren kann der Fachmann nun auf neue Marker zur Selektion mehrfach transformierter Pflanzenzellen zurückgreifen.

Die transgenen Pflanzenzellen können nach dem Fachmann bekannten Techniken zu ganzen Pflanzen regeneriert werden. Die durch Regeneration der erfindungsgemäßen transgenen Pflanzenzellen erhältlichen Pflanzen sind ebenfalls Gegenstand der vorliegenden Erfindung. Ferner sind Gegenstand der Erfindung Pflanzen und Pflanzengewebe, die die oben beschriebenen transgenen Pflanzenzellen enthalten. In Abhängigkeit des gewählten Promotors (z.B. 35S CaMV) sind auch die adulten Pflanzen resistent gegen 2-DOG und können durch Gabe dieser Verbindung selektioniert werden. Bei Verwendung von z.B. gewebespezifischen Promotoren entfällt diese Möglichkeit und der Fachmann kann z.B. auf molekularbiologische Methoden wie PCR zurückgreifen, um diese Pflanzen zu identifizieren. Auf der anderen Seite kann der Fachmann natürlich auch, z.B. nach Selbstung oder Rückkreuzung gegen den Elter, Samen solcher Pflanzen auf 2-DOG enthaltende Medien auslegen und anhand der Keimfähigkeit dieser Samen oder Überleben der Pflanzen in einem späteren Stadium der Entwicklung (abhängig vom gewählten Promotor) rückschließen, ob die Pflanzen transgen sind oder nicht. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen wie Weizen, Gerste, Reis, Raps, Erbse, Mais, Zuckerrübe, Zuckerrohr oder Kartoffel.
Die Erfindung betrifft ebenfalls Vermehrungsmaterial und Ernteprodukte der erfindungsgemäßen Pflanzen, beispielsweise Früchte, Samen, Knollen, Wurzelstöcke, Sämlinge, Stecklinge etc.

Wie oben bereits dargelegt, stellt die vorliegende Erfindung rekombinante DNA-Moleküle und Vektoren zur Verfügung, die als Selektionsmarker in Pflanzenzellen zur Selektion transformierter Pflanzenzellen geeignet sind sowie davon abgeleitete transgene Pflanzen, Pflanzenzellen und/oder Gewebe. So betrifft die vorliegende Erfindung auch die Verwendung eines erfindungsgemäßen rekombinanten DNA-Moleküls oder eines erfindungsgemäßen Vektors zur Herstellung von transgenen Pflanzen, Pflanzenzellen und/oder Gewebe sowie deren Nutzung als selektierbare Marker in pflanzlicher Zell- und Gewebekultur und/oder Pflanzenzüchtung.

### Die Figuren zeigen:

### Figur 1

Möglicher Mechanismus der Wachstumsinhibierung durch 2-Desoxyglucose (2-DOG) und Aufhebung der toxischen Wirkung durch Expression einer 2-DOG-6-P Phosphatase.

### Figur 2

A. Kultivierung von Tabak- und Kartoffelblattscheiben auf 2-DOG-haltigem MS-Medium. TOB, Nicotiana tabacum Var. Samsun NN; POT, Solanum tuberosum var. Solara; A, E: Kontrolle MS-Medium ohne Zusatz von 2-Desoxyglucose; B, F: MS-Medium mit 0.05 % 2-DOG; C, G: MS-Medium mit 0.1 % 2-DOG; D, H. MS-Medium mit 0.5 % 2-DOG.

B. Kultivierung von Erbsen-, Raps- und Weizenblattscheiben auf 2-DOG-haltigem MS-Medium. R, Raps; P, Erbse; W, Weizen. 0.0, 0.1 und 0.5: zugesetzte 2-DOG Konzentration in %.

### Figur 3

Schematische Darstellung der PCR-Amplifikation der 2-DOG-6-P Phosphatase aus Saccharomyces cerevisiae Stamm 288C

### Figur 4

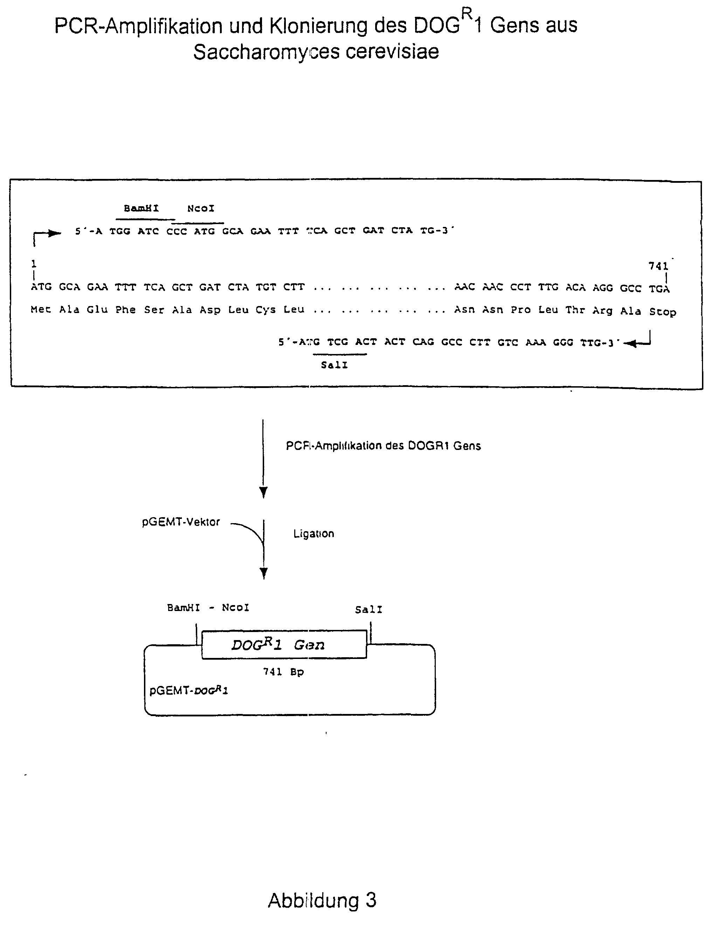

Pflanzliche Expressionskassette zur Überexpression des DOG^{R}1 Gens aus Hefe (*Saccharomyces cerevisiae* Stamm S288C) in transgenen Pflanzen Fragment A (529 bp): Beinhaltet den 35S Promotor des Cauliflower-Mosaik-Virus (CaMV). Es enthält ein Fragment, welches die Nukleotide 6909 bis 7437 des CaMV umfaßt.
Fragment B: DOG^{R}1 Geh aus Hefe, welches als BamHI/SaII Fragment aus dem Plasmid pGEMT-DOG^{R}1 isoliert wurde (s. Abbildung 3).
Fragment C (192 bp): Enthält das Polyadenylierungssignal des Gens 3 der T-DNA des Ti-Plasmids pTiACH5. Die Fragmente A bis C wurden über EcoRI/HindIII in den binären Vektor BIN19 (Bevan (1984) Nucl. Acid Res, 12, 8711) cloniert.

### Figur 5

Nachweis des DOG^{R}1-Gens mittels PCR-Amplifikation in genomischer DNA der transgenen Tabakpflanzen.

Spuren 1 und 14, DNA-Längenstandard; Spuren 2-10, unabhängige Transformanden; Spur 11, untransformierte Kontrollpflanze; Spur 12, Positivkontrolle (Plasmid pGEMT-DOG^{R}1); Spur 13, Wasserkontrolle.

### Figur 6

Nachweis der Expression des chimären DOG^{R}1-Gens in 2-DOG resistenten Tabakpflanzen mittels RNA-Analyse.
Northern-Analyse transgener Tabakpflanzen der Linie 35S-DOG. Gesamt RNA wurde aus Tabakblättern isoliert, gelelektrophoretisch aufgetrennt und auf eine Nylonmembran übertragen. Die Hybridisierung erfolgte mit der radioaktiv markierten Kodierregion des DOG^{R}1 Gens. Pro Spur sind 20 µg RNA aufgetragen. Spuren 1-9, unabhängig transformierte Pflanzen der Linie 35S-DOG; Spur 10, untransformierte Kontrolle.

### Figur 7

Nachweis der durch das DOG^{R}1-Gen vermittelten Resistenz in der Nachkommenschaft transgener Tabakpflanzen.
Samen von Nicotiana tabaccum Var. Samsun NN wurden sterilisiert und auf MS-Medium mit 0,05% 2-DOG ausgelegt. A: vier Wochen alte Keimlinge einer untransformierten Pflanze; B: vier Wochen alte Keimlinge einer das DOG^{R} 1-Gen unter der Kontrolle des 35S Promotors exprimierenden Pflanze

### Figur 8

Nachweis der durch das DOG^{R}1-Gen vermittelten Resistenz in Sprossen transgener Kartoffelpflanzen.
Sprosse von Kartoffelpflanzen Solanurn tuberosum var. Solara wurden auf 0,05% 2-DOG enthaltendes MS-Medium gesetzt. A: fünf Wochen alter Spross einer untransformierten Pflanze; B: fünf Wochen alter Spross einer das DOG^{R}1-Gen unter der Kontrolle des 35S Promotors exprimierenden Pflanze

### Figur 9

Pflanzliche Expressionskassette zur Überexpression des DOG^{R}1-Gens aus Hefe (Saccharomyces cerevisiae Stamm 288C) in transgenen Erbsenpflanzen.
Fragment A (529 bp): Beinhaltet den 35S Promotor des Cauliflower-Mosaik-Virus (CaMV). Es enthält ein Fragment, welches die Nukleotide 6909 bis 7437 des CaMV umfaßt
Fragment B (73 bp): untranslatierter Translationsenhancer aus dem Tabak Mosaik Virus U1 (Gallie (1987) Nucl. Acids Res. 15, 8693)
Fragment C: DOG^{R}1-Gen aus Hefe
Fragment D (192 bp): Enthält das Polyadenylierungssignal des Gens 3 der T-DNA des Ti-Plasmids pTiACH5
Die Fragmente A bis D wurden über EcoRI/HindIII in den binären Vektor pGPTV (Becker (1992) PMB 20, 1195) kloniert

### Figur 10

Nachweis der durch das das DOG^{R}1-Gen vermittelten Resistenz in Erbsenpflanzen. Kallus von Erbsenpflanzen Pisum sativum wurden auf 0,075% 2-DOG enthaltendes B5-Medium gelegt. A: Kallus untransformierter Pflanze auf 0,075% 2-DOG; B: Kallus von das DOG^{R}1-Gen unter der Kontrolle des 35S Promotors exprimierenden Pflanzen auf 0,075% 2-DOG

### In den Beispielen verwendete Methoden:

### 1. Allgemeine Clonierungsverfahren

Clonierungsverfahren wie z.B.: Restriktionsspaltungen, DNA-Isolierung, Agarose-Gelelektrophorese, Reinigung von DNA-Fragmenten, Transfer von Nukleinsäuren auf Nitrozellulose und Nylonmembranen, Verknüpfen von DNA-Fragmenten, Transformation von E.coli Zellen, Anzucht von Bakterien, Sequenzanalyse rekombinanter DNA wurden nach Sambrook (Cold Spring Harbor Laboratory Press (1989); ISBN 0-87969-309-6) beschrieben durchgeführt. Die Transformation von Agrobacterium tumefaciens wurde entsprechend der Methode von Höfgen und Willmitzer (Nucl. Acid Res. (1988) 16, 9877) ausgeführt. Die Anzucht der Agrobacterien erfolgte in YEB Medium (Vervliet, Gen. Virol. (1975) 26, 33).

### 2. Bakterienstämme

E.coli (XL-1 Biue) Bakterien wurden durch die Firma Stratagene bezogen. Der zur Pflanzentransformation eingesetzte Agrobacterium Stamm (C58C1 mit dem Plasmid pGV 3850kan) wurde von Deblaere (1985, Nucl. Acid Res. 13, 4777) beschrieben.

### 3. Analyse von Gesamt-RNA aus pflanzlichen Geweben

Pflanzliche Gesamt-RNA wurde nach der Methode von Logemann (1987, Analytical Biochem. 163, 16) isoliert und in Formaldehyd-haltigen Agarosegelen aufgetrennt (Lehrach (1977) Biochem. 16, 4743). Ein Kappilar-Transfer auf Nylon-Membranen (Gene Screen, NEN) erfolgte in 20 x SSC (1.5 M NaCI, 150 mM Natriumcitrat) über Nacht. Nach zweistündiger Prähybridisierung in Hybridisierungspuffer (500 mM Natriumphosphat (pH 7.2), 7 % SDS, 1 % Rinderserumalbumin, 200 µg Heringssperma DNA, 1 mM EDTA), erfolgte die Hybridisierung mit der radioaktivmarkierten DOG^{R}1-Sonde bei 55°C für 16 Stunden. Als Sonde wurde die DOG^{R}1 Codierregion aus dem Plasmid pGEMT isoliert und mittels High Prime Kit (Boehringer) in Anwesenheit von α-³²P-dCTP radioaktiv markiert. Anschließend wurden die Filter unter folgenden Bedingungen gewaschen: 20 Minuten bei 55°C in 6 x SSC, 0.1 % SDS und 20 Minuten bei 55°C in 4 x SSC, 0.1 % SDS.

### 4. Bestimmung von DOG-6-P in Pflanzenextrakten

Zum Nachweis der 2-DOG-6-P Phosphatase Aktivität in den transgenen Pflanzen wurden Blattscheiben (ca. 100 mg Frischgewicht) in 300 mM 2-DOG Lösung für 24 Stunden im Dunkeln inkubiert. Anschließend wurden die Blattscheiben 1 Minute mit Wasser gewaschen und in flüssigem Stickstoff eingefroren. Zur Extraktion der Metabolite wurde das Pflanzenmaterial in auf Trockeneis vorgekühken Mörsem zu einem feinen Pulver homogenisiert und mit 1.5 ml 16 % (w/v) Trichloressigsäure (TCA) in Diethylether (vorgekühlt auf 4°C) versetzt. Anschließend wurden die Homogenate 15 Minuten auf Trockeneis inkubiert. Durch Zugabe von 0.8 ml 16 % TCA (w/v), 5 mM EGTA wurden die Metabolite gelöst. Nach Transfer der Homogenate in Eppendorfreaktionsgefäße wurden diese 3 Stunden bei 4°C inkubiert. Anschließend wurden die Proben 5 Minuten bei 15 000 UpM und 4°C in einer Biofuge 15R (Heraeus) zentrifugiert. Die wäßrige Phase wurde in Eppendorfreaktionsgefäße überführt und viermal mit wassergesättigtem Ether gewaschen. Anschließend wurden die Proben mit 5 M KOH/1 M Triethanolamin-Gemisch neutralisiert und für 1 Minute mit Stickstoff begast. Die so vorbereiteten Proben wurden mittels HPLC analysiert. Für die Analyse wurde ein HPLC System der Firma Dionex verwendet, welches mit einer PA-1 (4 x 250 mm) Säule und einem gepulsten elektrochemischen Detektor ausgestattet war. Vor der Injektion wurden die Proben für 2 Minuten bei 13 000 UpM abzentrifugiert. Metabolite wurden anschließend mit einem 50-minütigen konkaven Gradienten (Kurve 9) von 1 mM bis 500 mM Natriumacetat nach 40 Minuten bei 10 mM NaOH und einer Durchflußrate von 1 ml/min. eluiert. Isokratische Elution wurde für weitere 5 Minuten durchgeführt. Zur Identifizierung und Quantifizierung von 2-DOG-6-P wurde als Standard 2-DOG-6-P der Firma Sigma verwendet.

Die Beispiele erläutern die Erfindung.

### Beispiel 1: Nachweis der Toxizität von 2-Desoxyglucose (2-DOG) für Pflanzenzellen

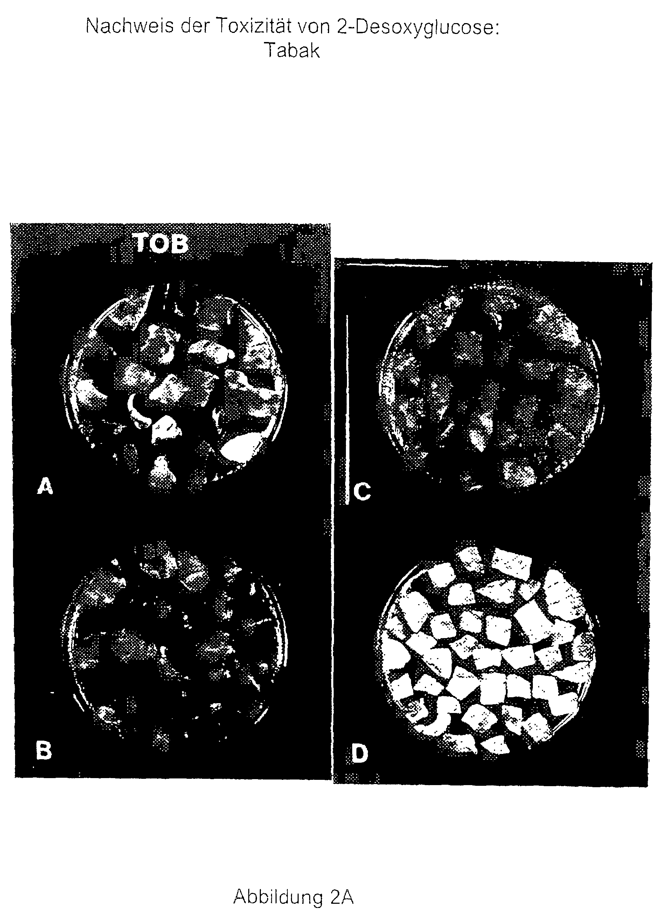

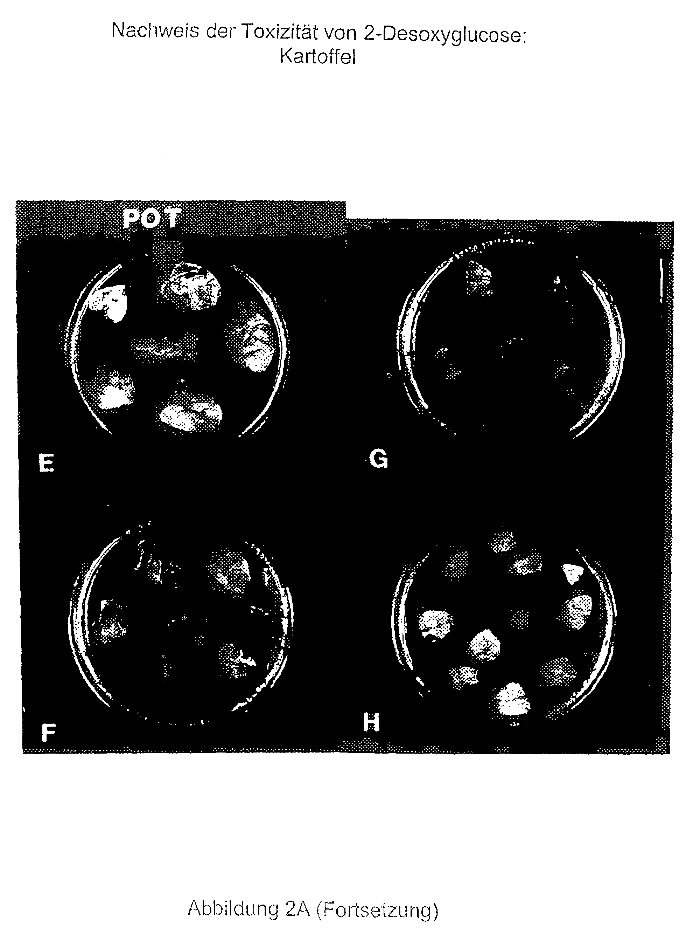

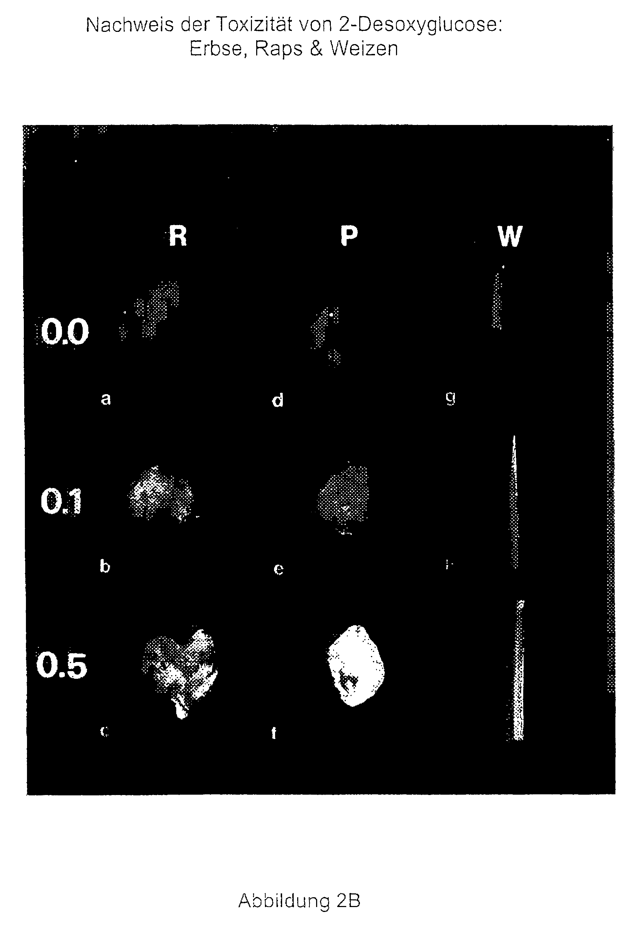

Zum Nachweis der Toxizität von 2-DOG wurden Blattstücke (ca. 1 cm²) von sterilen Tabak- und Kartoffelpflanzen auf Murashige und Skoog-Medium (Murashige & Skoog (1962) Physiol. Plant. 15, 473), dem 0.01 bis 0.5 % 2-DOG zugesetzt wurde, für zwei Wochen kultiviert. Wie in Abbildung 2 gezeigt, führt Zusatz von 2-DOG (Abb. 2, B-D und F-H) zum Absterben der Blattscheiben.

### Beispiel 2: PCR-Amplifikation der 2-DOG-6-F Fhospliatase aus Saccharomyces cerevisiae Stamm S288C

Die Clonierung der DOG^{R}1 Codierregion erfolgte mittels der Polymerase Kettenreaktion (**P**olymerase **C**hain **R**eaction, PCR). Als Matrizenmaterial diente genomische Hefe DNA, die aus Saccharomyces cerevisiae Stamm S288C nach Standardprotokoll isoliert wurde. Die Amplifizierung erfolgte unter Verwendung der spezifischen Primer DOG^{R}1-1 (5'-ATGGATCCCCATGGCAGAATTTTCAGCTGATCTATG-3'; SEQ ID NO:3) und DOG^{R}1-2 (5' ATGTCGACTACTCAGGCCCTTGTCAAAGGGTTG-3'; SEQ ID NO:4), die von einer publizierten Sequenz abgeleitet wurden (Sanz (1994) Yeast 10, 1195-1202). Primer 1 umfaßt die Basen 1 bis 26 und Primer 2 die Basen 720 bis 741 der Codierregion des DOG^{R}1 Gens. Zur Clonierung der amplifizierten DNA in pflanzliche Expressionsvektoren tragen die Primer zusätzlich folgende Restriktionsschnittstellen: Primer 1, BamHI und Ncol; Primer 2, Sall. Die Clonierungsstrategie ist in Abbildung 3 dargestellt. Das PCR Reaktionsgemisch (50µl) enthielt chromosomale Hefe DNA (1µg), Primer 1 und 2 (jeweils 1µg), 10 mM Tris-HCl (pH 8.8 bei 25°C), 3.5 mM MgCl₂, 50 mM KCI, 0.1 % Triton X-100, 200 µM dNTPs (dATP, dCTP, dGTP, dTTP) und 2 Units PrimeZyme DNA Polymerase (Biometra). Vor Zugabe der Polymerase wurde das Gemisch 10 Minuten auf 94°C erhitzt. Die Polymerisierungschritte (60 Zyklen) wurden in einem automatischen "Thermocycler" (Perkin Elmer) nach folgendem Programm durchgeführt: Denaturierung bei 94°C (1 Minute), Anlagerung der Primer bei 40°C (1 Minute), Polymerase-Reaktion bei 72°C (1 Minute). Das erhaltene Fragment wurde in den Vektor pGEMT (Promega Corp., 2800 Woods Hollow Road, Madison, Wl 53711-5399, USA) cloniert, wodurch das Plasmid pGEMT-DOG^{R}1 erhalten wurde. Die Identität der amplifizierten DNA wurde mittels Sequenzanalyse verifiziert.

### Beispiel 3: Herstellung von Plasmid p35S-DOG^{R}1

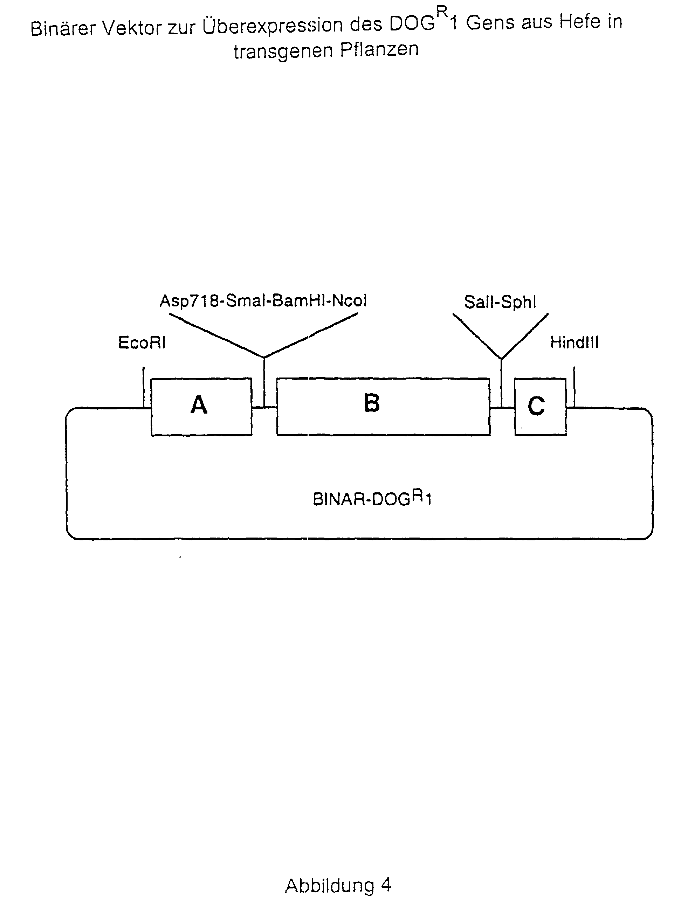

Eine DNA-Sequenz, die für eine 2-DOG-6-P Phosphatase codiert, wurde aus dem Plasmid pGEMT-DOG^{R}1 isoliert und mit dem 35S-Promotor des Cauliflower-Mosaik-Virus, der eine konstitutive Expression in transgenen Pflanzen bewirkt, sowie einem pflanzlichen Transkriptions-Terminationssignal versehen. Das pflanzliche Transkriptions-Terminationssignal beinhaltet das 3'-Ende der Polyadenylierungsstelle des Octopin-Synthase Gens. Das Plasmid p35S-DOG^{R}1 beinhaltet drei Fragmente A, B und C, die in die Schnittstellen für Restriktionsenzyme des Polylinkers von pUC 18 cloniert wurden (s. Abbildung 4). Das Fragment A beinhaltet den 35S Promotor des Cauliflower-Mosaik-Virus (CaMV). Es enthält ein Fragment, welches die Nukleotide 6909 bis 7437 des CaMV umfaßt (Franck (1980) Cell 21, 285) und wurde als EcoRl-Kpnl Fragment aus dem Plasmid pDH51 (Pietrzak (1986) Nucleic Acid Fes. 14, 5857) isoliert und zwischen die EcoRI-Kpnl Schnittstellen des Plasmids pUC18 cloniert.
Das Fragment B enthält die DOG^{R}1 Codierregion, die als BamHI-SaII Fragment aus dem Plasmid pGEMT-DOG^{R}1 (s. Abb. 3) isoliert und zwischen die BamHI-SaII Schnittstellen des Polylinkers von pUC18 cloniert wurde.
Das Fragment C enthält das Polyadenylierungssignal des Gens 3 der T-DNA des Ti-Plasmids pTiACH5 (Gielen (1984) EMBO J. 3, 835), Nukleotide 11749 - 11939, welches als PvuII-HindIII Fragment aus dem Plasmid pAGV 40 (Herrera-Estrella (1983) Nature 303, 209) isoliert worden ist und nach Addition von Sphl-Linkern an die Pvull-Schnittstelle zwischen SphI-HindIII Schnittstellen des Polylinkers von pUC 18 cloniert war.
Das chimäre Gen wurde anschließend als EcoRI-HindIII Fragment zwischen die EcoRI-HindIII Schnittstellen des Plasmids pBIN19 (Bevan (1984) Nucleic. Acid Res. 12, 8711) cloniert.

### Beispiel 4: Selektion von DOG^{R}1 transformierten Pflanzenzellen und Pflanzenregeneration auf 2-DOG enthaltendem Medium

10 ml einer unter Selektion gewachsenen Übernachtkultur von Agrobacterium tumefaciens wurden abzentrifugiert, der Überstand verworfen, und die Bakterien in gleichen Volumen Antibiotika-freien Medium resuspendiert. In einer sterilen Petrischale wurden Blattscheiben steriler Nicotiana tabacum Var. Samsun NN Pflanzen (ca. 1cm²), denen die Mittelrippe entfernt wurde, in dieser Bakterienkultur gebadet. Anschließend wurden die Blattscheiben in Petnschalen, die MS-Medium mit 2% Saccharose und 0.8% Bacto Agar enthalten, dicht ausgelegt. Nach 2tägiger Inkubation im Dunkeln bei 25°C wurden sie auf MS-Medium übertragen, welches 0.05% 2-DOG, 400 mg/l β-Bactyl, 1 mg/l Benzaminopurin (BAP), 0.2 mg/l Naphthylessigsäure (NAA), 1.6 % Glucose und 0.8% Bacto Agar enthielt. Nach Callusbildung wurden die Blattscheiben bis zur Sproßbildung auf MS-Medium übertragen, welches 0.02% 2-DOG, 400 mg/l β-Bactyl, 1 mg/l Benzaminopurin (BAP), 0.2 mg/l Naphthylessigsäure (NAA), 1.6 % Glucose und 0.8% Bacto Agar enthielt. Wachsende Sprosse wurden auf hormonfreies MS-Medium mit 200 mg/l β-Bactyl und 2 % Saccharose zur Wurzelbildung überführt. Anschließend wurden bewurzelte Sprosse in Erdkultur überführt und im Gewächshaus kultiviert.

### Beispiel 5: Nachweis des chimären DOG^{R}1-Gens in den transformierten Pflanzen mittels PCR

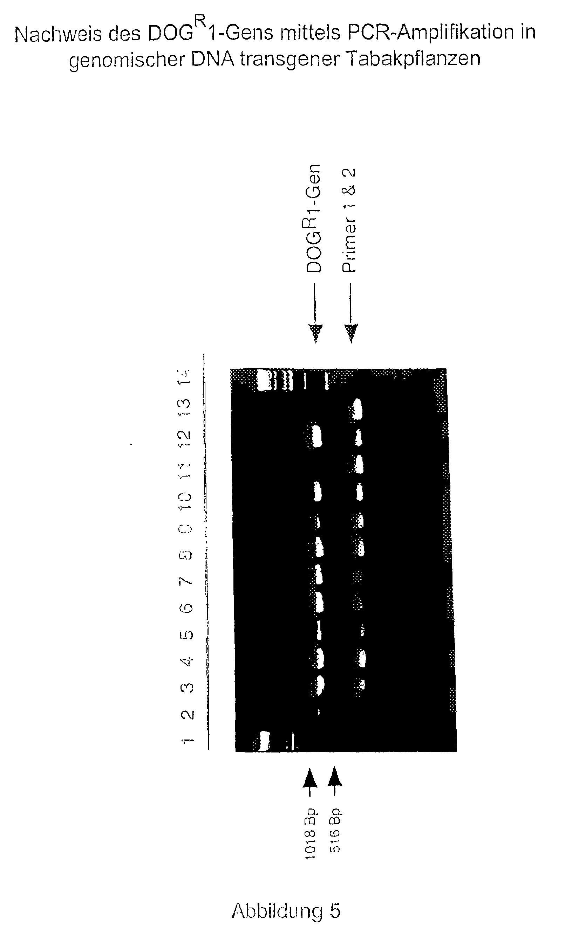

Zum Nachweis der erfolgreichen Transformation wurde genomische DNA der transgenen Tabakpflanzen nach der Methode von Rogers und Bendich (Plant Mol. Biol. (1985) 5, 69) isoliert und das chimäre DOG^{R}1 Gen mittels PCR-Amplifikation nachgewiesen. Das PCR Reaktionsgemisch (50µl) enthielt genomische Tabak DNA (1µg), Primer 1 und 2 (jeweils 1µg), 10 mM Tris-HCI (pH 8.8 bei 25°C), 3.5 mM MgCl₂, 50 mM KCl, 0.1 % Triton X-100, 200 µM dNTPs (dATP, dCTP, dGTP, dTTP) und 2 Units PrimeZyme DNA Polymerase (Biometra). Vor Zugabe der Polymerase wurde das Gemisch 10 Minuten auf 94°C erhitzt. Die Polymerisierungschritte (40 Zyklen) wurden in einem automatischen "Thermocycler" (Perkin Elmer) nach folgendem Programm durchgeführt: Denaturierung bei 94°C (1 Minute), Anlagerung der Primer bei 40°C (1 Minute), Polymerase-Reaktion bei 72°C (1 Minute). Anschließend wurde ein Aliquot der PCR-Reaktion in einem Agarosegel aufgetrennt. Wie in Abbildung 5 dargestellt, wird unter Verwendung genomischer DNA transgener Pflanzen als Matrize ein ca. 740 Basenpaar großes DNA-Fragment amplifiziert, welches unter Verwendung genomischer DNA von untransformierten Kontrollpflanzen nicht nachweisbar ist.

### Beispiel 6: Nachweis der Expression des chimären DOG^{R}1-Gens in 2-DOG-resistenten Tabakpflanzen mittels RNA-Analyse

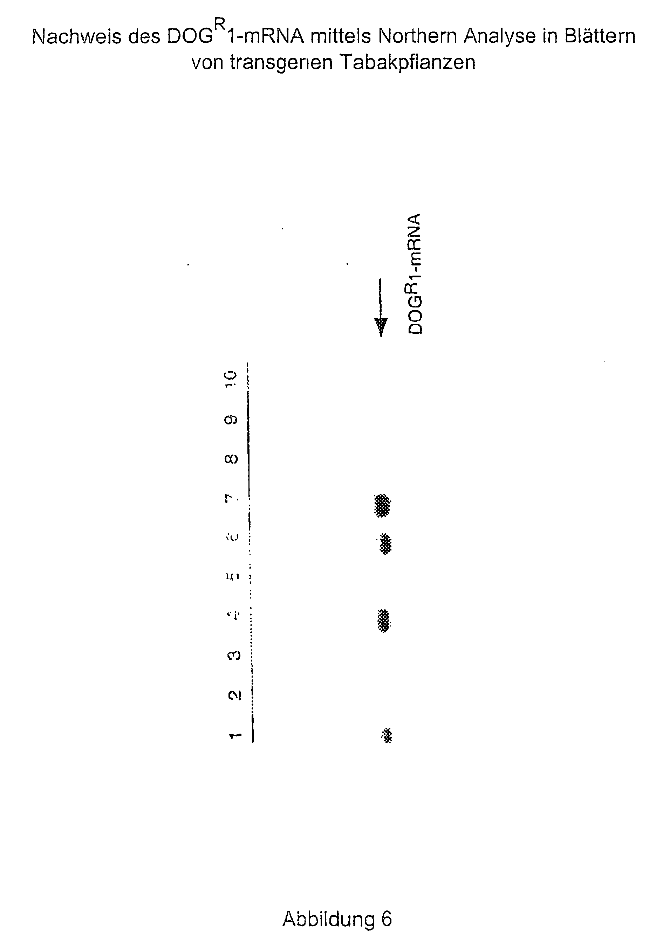

Die Expression der 2-DOG-6-P Phosphatase in den regenerierten Pflanzen wurde mittels RNA-Analysen verifiziert. Hierzu wurden Blattproben der ins Gewächshaus überführten Pflanzen geerntet, Gesamt-RNA extrahiert, elektrophoretisch aufgetrennt, auf Nylonmembranen übertragen und mit der Codierregion des DOG^{R}1 Gens hybridisiert. Wie in Abbildung 6 dargestellt, konnte in den auf 2-DOG regenerierten Tabakpflanzen die 2-DOG-6-P Phosphatase mRNA nachgewiesen werden. Eine Kreuzreaktion mit untransformierten Tabakpflanzen konnte nicht beobachtet werden.

### Beispiel 7: In vivo Nachweis der 2-DOG-6-P Phosphatase Aktivität

Der Nachweis der in vivo Funktionalität der 2-DOG-6-P Phosphatase wurde wie folgt durchgeführt: 0.78 cm² große Blattscheiben von untransformierten Tabakpflanzen und den Transformanden 35S-DOG-3, 4, 8, 9 und 11 wurden 24 Stunden in einer 300 mM 2-DOG Lösung im Dunkeln inkubiert. Zur Detektion des gebildeten 2-DOG-6-P wurden die phosphorylierten Intermediate isoliert und mittels HPLC aufgetrennt. Wie in Tabelle 1 dargestellt, führt die Expression der 2-DOG-6-P Phosphatase zu einer Verminderung der 2-DOG-6-P Akkumulation.

**Tabelle 1:**

| Nachweis von 2-DOG-6-P in Blattscheiben von transgenen und untransformierten Tabakpflanzen | | |
|---|---|---|
| Genotyp | [µmol m⁻²] 2-DOG-6-P | [% der Kontrolle] 2-DOG-6-P |
| Kontrolle | 576 ± 40 | 100 |
| 355-DOG-3 | 170 ± 26 | 31 ± 6.0 |
| 35S-DOG-4 | 223 ± 18 | 40 ± 6.0 |
| 35S-DOG-8 | 103 ± 11 | 18 ± 3.0 |
| 35S-DOG-9 | 250 ± 24 | 45 ± 7.0 |
| 35S-DOG-11 | 283 ± 12 | 50 ± 5.0 |

Legende: Kontrolle, untransformierte Nicotiana tabacum Var. Samsun NN Pflanze; 35S-DOG, transgene Pflanzen; die Daten entsprechen den Mittelwerten (n=4) ± Standardabweichung.

### Beispiel 8: Nachweis der durch das DOG^{R}1-Gen vermittelten Resistenz in der Nachkommenschaft transgener Tabakpflanzen

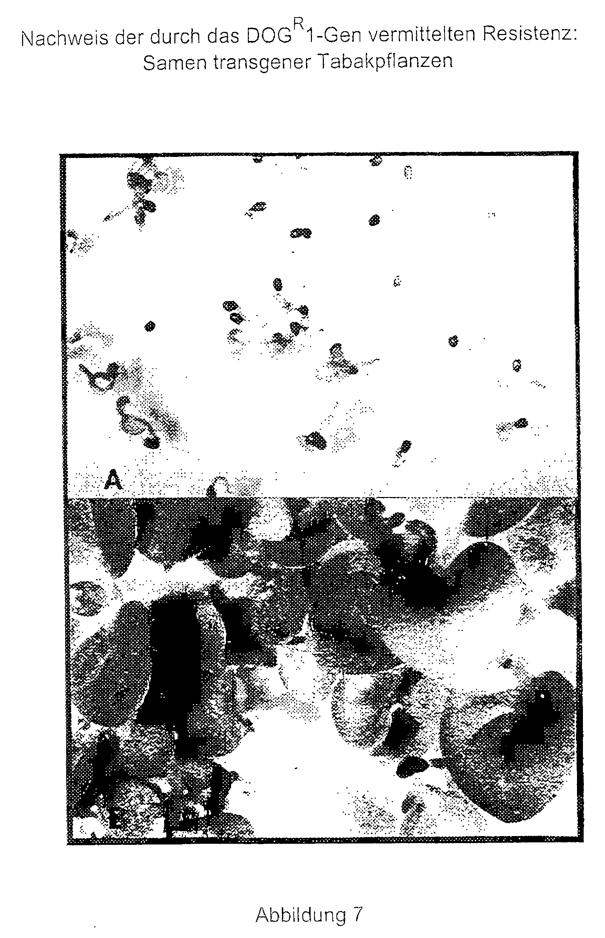

Zum Nachweis der Resistenz von Keimlingen die das DOG^{R}1-Gen exprimieren wurden Samen der in Beispiel 5 und 6 beschriebenen Tabakpflanzen sowie Samen untransformierter Tabakpflanzen sterilisiert und auf 0,05% 2-DOG enthaltendes MS-Medium ausgelegt. Wie in Abbildung 7 dargestellt, sind die Keimlinge die das DOG^{R}1-Gen unter der Kontrolle des 35S Promotors exprimieren nach vier Wochen vital, wohingegen die Keimlinge der untransformierten Pflanzen in einem frühen Entwicklungsstadium abgestorben sind.

### Beispiel 9: Nachweis der durch das DOG^{R}1-Gen vermittelten Resistenz in Sprossen von Kartoffelpflanzen

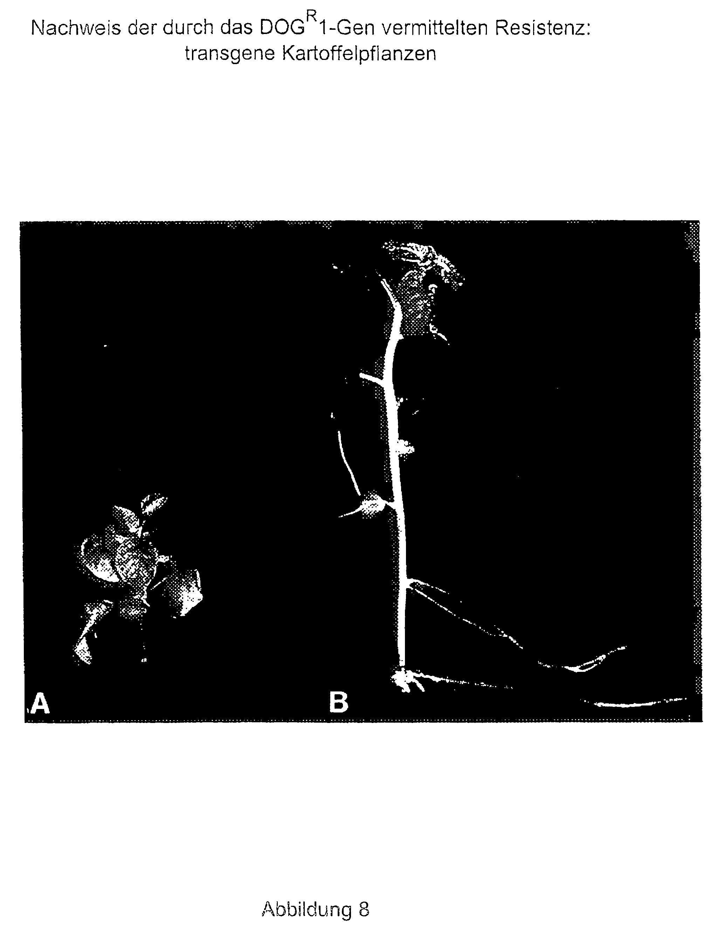

In Kartoffelpflanzen Solanum tuberosum var. Solara wurde durch Agrobakterien vermittelte Transformation das im Beispiel 3 beschriebenen Konstrukt übertragen. Die regenerierten Sprosse wurden auf selektionsfreiem Medium angezogen. Die Sprosspitzen wurden nach sechs Wochen überführt auf 0,05% 2-DOG enthaltendes MS-Medium. Wie in Abbildung 8 dargestellt, ist der Spross einer untransformierten Pflanze nach fünf Wochen nicht gewachsen, wohingegen der Spross, der das DOG^{R}1-Gen unter der Kontrolle des 35S Promotors exprimiert, Wurzel- und Stammwachstum zeigt.

### Beispiel 10: Herstellung des Plasmids p35S Omega-DOG^{R}1 zur Transformation von Erbse

Zur Transformation von Erbse Pisum sativum wurde in pUC18 ein Konstrukt erstellt, welches den 35S Promotor aus Cauliflower Mosaik Virus, einen untranslatierten Transkriptionsenhancer des Tabak Mosaik Virus U1 (Sonnewald (1992) Plant Journal 2 (4) 571), das DOG^{R}1-Gen und einen Transkriptionsterminator OCS enthält. Dieses Konstrukt wurde als EcoRI/HindIII Fragment in den binären Vektor pGPTV (Becker (1992) PMB 20, 1195) eingeführt.

### Beispiel 11: Selektion von DOG^{R}1 transformierten Erbsenzellen und Pflanzenregeneration auf 2-DOG enthaltendem Medium

Sterile unreife Samen von Erbse Pisum sativum wurden 2-3 Tage. in flüssigem auxin/ cytokininhaltigem Medium vorkultiviert. Danach wurden longitudinale Schnitte der Embryonenachse präpariert und für 1h in einer Übernachtkultur von Agrobakterium tumefaciens inkubiert. Die Explantate wurden danach auf festes Pflanzenmedium übertragen. Nach 4tägiger Inkubation auf modifiziertem B5-Medium im diffusen Licht bei 22°C wurden die Explantate gewaschen und auf festes auxin-/ cytokinin- und 0,075% 2-DOG haltiges Medium übertragen. Die Selektion erfolgte auf verschiedenen Wuchsstoffkonzentrationen mit einem permanenten Selektionsdruck von 0,075% 2-DOG. Regenerierte Sprosse wurden auf untransformierte Sämlinge gepfropft, in Erde überführt und im Gewächshaus kultiviert.

### Beispiel 12: Nachweis der durch das DOG^{R}1-Gen vermittelten Resistenz in Blättern von Erbsenpflanzen

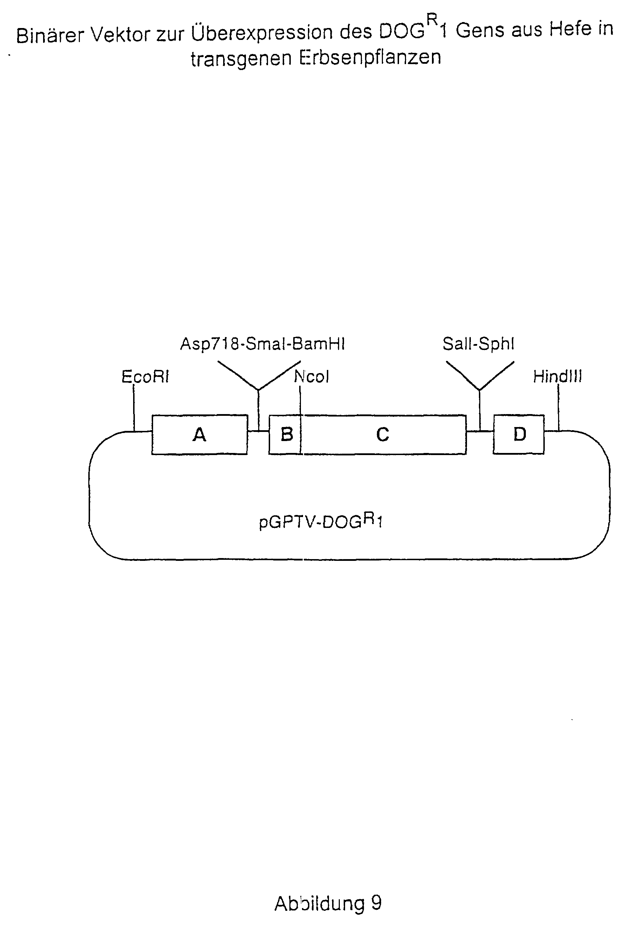

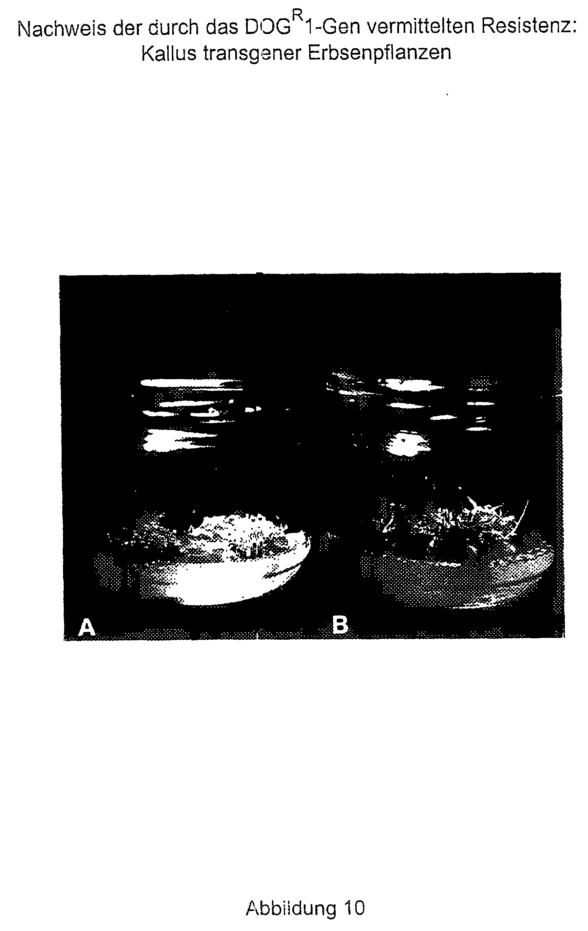

Auf Erbsenpflanzen Pisum sativurn wurde durch Agrobakterien vermittelte Transformation das im Beispiel 10 beschriebenen Konstrukt übertragen. Kallus transformierter und nicht transformierter Pflanzen wurden auf 0,075% 2-DOG enthaltendes Medium gelegt. Wie in Abbildung 10 dargestellt, entwickelt der Kallus untransformierter Pflanzen keine Sprosse, wohingegen der nach Transformation mit dem DOG^{R}1-Gen unter der Kontrolle des 35S Promotors entwickelte Kalius Sprosse bildet.

## Patentansprüche

1. Rekombinantes DNA-Molekül umfassend
(a) regulatorische Sequenzen eines in Pflanzen aktiven Promotors;
(b) operativ daran gebunden eine DNA-Sequenz, die ein Protein mit der biologischen Aktivität einer 2-Desoxyglucose-6-Phosphat (2-DOG-6-P) Phosphatase codiert; und
(c) operativ daran gebunden regulatorische Sequenzen, die als Transkriptions-Terminations und/oder Polyadenylierungssignale in Pflanzen dienen.

2. Rekombinantes DNA-Molekül nach Anspruch 1, wobei die DNA-Sequenz, die ein Protein mit der biologischen Aktivität einer 2-DOG-6-P Phosphatase codiert, ausgewählt ist aus der Gruppe bestehend aus
(a) DNA-Sequenzen, die eine Nucleotid-Sequenz umfassen, die die in SEQ ID NO. 2 angegebene Aminosäuresequenz codiert;
(b) DNA-Sequenzen, die die in SEQ ID NO. 1 angegebene Nucleotidsequenz umfassen;
(c) DNA-Sequenzen umfassend eine Nucleotidsequenz, die mit einem komplementären Strang der Nucleotid-Sequenz von (a) oder (b) hybridisiert;
(d) DNA-Sequenzen umfassend eine Nucleotidsequenz, die zu einer Nucleotidsequenz von (c) degeneriert ist, und
(e) DNA-Sequenzen, die ein Derivat, Analog oder Fragment einer Nucleotidsequenz von (a), (b), (c) oder (d) ist und für ein Protein codiert, das 2-DOG-6-P Phosphatase Aktivität besitzt.

3. Rekombinantes DNA-Molekül nach Anspruch 1 oder 2, wobei die DNA-Sequenz aus Hefe stammt.

4. Rekombinantes DNA-Molekül nach einem der Ansprüche 1 bis 3, wobei der Promotor der 35S CaMV Promotor ist.

5. Vektor umfassend ein rekombinantes DNA-Molekül nach einem der Ansprüche 1 bis 4.

6. Vektor nach Anspruch 5, der mindestens ein weiteres rekombinantes DNA-Molekül enthält.

7. Vektor nach Anspruch 6, wobei das weitere rekombinante DNA-Molekül eine DNA-Sequenz enthält, die ein Peptid, Protein, Antisense-, Sense-RNA, virale RNA oder Ribozym codiert.

8. Wirtszelle, enthaltend ein rekombinantes DNA-Molekül nach einem der Ansprüche 1 bis 4 oder einen Vektor nach einem der Ansprüche 5 bis 7.

9. Kit umfassend ein rekombinantes DNA-Molekül nach einem der Ansprüche 1 bis 4 oder einen Vektor nach einem der Ansprüche 5 bis 7 und gegebenenfalls 2-Desoxyglucose oder eine zu 2-Desoxyglucose funktionell äquivalente chemische Verbindung.

10. Verfahren zur Selektion transformierter Pflanzenzellen, das die folgenden Schritte umfaßt:
(a) Gewinnung von Pflanzenzellen;
(b) Einführung eines rekombinanten DNA-Moleküls nach einem der Ansprüche 1 bis 4 oder eines Vektors nach einem der Ansprüche 5 bis 7 in diese Pflanzenzellen; und
(c) Selektion der erfolgreich transformierten Pflanzenzellen auf 2-Desoxyglucose-enthaltenden Medien oder auf Medien, die eine zu 2-Desoxyglucose funktionell äquivalente chemische Verbindung enthalten.

11. Verfahren nach Anspruch 10, wobei der Vektor nach einem der Ansprüche 5 bis 7 mittels Agrobacterium tumefaciens in Pflanzenzellen übertragen wird.

12. Verfahren nach Anspruch 10, wobei das rekombinante DNA-Molekül nach einem der Ansprüche 1 bis 4 oder der Vektor nach einem der Ansprüche 5 bis 7 mittels Partikel-Bombardment in Pflanzenzellen übertragen wird.

13. Transgene Pflanzenzelle enthaltend ein rekombinantes DNA-Molekül nach einem der Ansprüche 1 bis 4 oder einen Vektor nach einem der Ansprüche 5 bis 7 oder hergestellt nach dem Verfahren nach einem der Ansprüche 10 bis 12.

14. Pflanzenzelle nach Anspuch 13, die mindestens ein weiteres Fremdgen enthält.

15. Pflanzengewebe umfassend Pflanzenzellen nach Anspruch 13 oder 14 oder hergestellt nach dem Verfahren nach einem der Ansprüche 10 bis 12.

16. Transgene Pflanze enthaltend eine Pflanzenzelle nach Anspruch 13 oder 14 oder hergestellt nach dem Verfahren nach einem der Ansprüche 10 bis 12.

17. Ernteprodukte der Pflanze nach Anspruch 16 umfassend Pflanzenzellen nach Anspruch 13 oder 14.

18. Vermehrungsmaterial der Pflanzen nach Anspruch 16, umfassend Pflanzenzellen nach Anspruch 13 oder 14.

19. Verwendung eines DNA-Moleküls, das eine DNA-Sequenz umfaßt wie sie in einem der Ansprüche 1 bis 3 definiert ist, eines rekombinanten DNA-Moleküls nach einem der Ansprüche 1 bis 4 oder eines Vektors nach einem der Ansprüche 5 bis 7 zur Herstellung von transgenen Pflanzen, Pflanzenzellen und/oder Gewebe.

20. Verwendung eines DNA-Moleküls, das eine DNA-Sequenz umfaßt wie sie in einem der Ansprüche 1 bis 3 definiert ist, eines rekombinanten DNA-Moleküls nach einem der Ansprüche 1 bis 4 oder eines Vektors nach einem der Ansprüche 5 bis 7 als selektierbarer Marker in pflanzlicher Zell- und Gewebekultur und/oder Pflanzenzüchtung.

## Claims

1. Recombinant DNA molecule comprising
(a) regulatory sequences of a promoter active in plants;
(b) operably linked thereto a DNA sequence encoding a protein with the biological activity of a 2-deoxyglucose-6-phosphate (2-DOG-6-P) phosphatase; and
(c) operably linked thereto regulatory sequences which serve as transcription termination and/or polyadenylation signals in plants.

2. The recombinant DNA molecule of claim 1, wherein the DNA sequence which encodes a protein with the biological activity of a 2-DOG-6-P phosphatase is selected from the group consisting of
(a) DNA sequences comprising a nucleotide sequence which encodes the amino acid sequence indicated in SEQ ID NO. 2;
(b) DNA sequences comprising the nucleotide sequence indicated in SEQ ID NO. 1;
(c) DNA sequences comprising a nucleotide sequence which hybridizes to a complementary strand of the nucleotide sequence of (a) or (b);
(d) DNA sequences comprising a nucleotide sequence which is degenerate to a nucleotide sequence of (c), and
(e) DNA sequences being a derivative, analogue or fragment of a nucleotide sequence of (a), (b), (c) or (d) and encoding a protein possessing 2-DOG-6-P phosphatase activity.

3. The recombinant DNA molecule of claim 1 or 2, wherein the DNA sequence is derived from yeast.

4. The recombinant DNA molecule of any one of claims 1 to 3, wherein the promoter is the 35S CaMV promoter.

5. Vector comprising a recombinant DNA molecule of any one of claims 1 to 4.

6. The vector of claim 5 which contains at least one further recombinant DNA molecule.

7. The vector of claim 6, wherein the further recombinant DNA molecule contains a DNA sequence which encodes a peptide, protein, antisense-, sense-RNA, viral RNA or ribozyme.

8. Host cell containing a recombinant DNA molecule of any one of claims 1 to 4 or a vector of any one of claims 5 to 7.

9. Kit comprising a recombinant DNA molecule of any one of claims 1 to 4 or a vector of any one of claims 5 to 7 and optionally 2-deoxyglucose or a chemical compound functionally equivalent to 2-deoxyglucose.

10. Process for selecting transformed plant cells, comprising the following steps:
(a) obtaining plant cells;
(b) introducing a recombinant DNA molecule of any one of claims 1 to 4 or a vector of any one of claims 5 to 7 into these plant cells; and;
(c) selecting the successfully transformed plant cells on 2-deoxyglucose-containing media or on media containing a chemical compound which is functionally equivalent to 2-deoxyglucose.

11. The process of claim 10, wherein the vector of any one of claims 5 to 7 is transferred to plant cells via Agrobacterium tumefaciens.

12. The process of claim 10, wherein the recombinant DNA molecule of any one of claims 1 to 4 or the vector of any one of claims 5 to 7 is transferred to plant cells by particle bombardment.

13. Transgenic plant cell containing a recombinant DNA molecule of any one of claims 1 to 4 or a vector of any one of claims 5 to 7 or produced according to the process of any one of claims 10 to 12.

14. Plant cell of claim 13, which contains at least one further foreign gene.

15. Plant tissue comprising plant cells of claim 13 or 14 or produced according to the process of any one of claims 10 to 12.

16. Transgenic plant containing a plant cell of claim 13 or 14 or produced according to the process of any one of claims 10 to 12.

17. Harvest products of the plant of claim 16 comprising plant cells of claim 13 or 14.

18. Propagation material of the plants of claim 16 comprising plant cells of claim 13 or 14.

19. Use of a DNA molecule which comprises a DNA sequence as defined in any one of claims 1 to 3, of a recombinant DNA molecule of any one of claims 1 to 4 or of a vector of any one of claims 5 to 7 for producing transgenic plants, plant cells and/or tissue.

20. Use of a DNA molecule which comprises a DNA sequence as defined in any one of claims 1 to 3, of a recombinant DNA molecule of any one of claims 1 to 4 or of a vector of any one of claims 5 to 7 as selectable marker in plant cell and tissue culture and/or plant breeding.

## Revendications

1. Molécule d'ADN recombinant, comprenant
(a) des séquences régulatrice, d'un promoteur actif chez des plantes ;
(b) fonctionnellement liée à celles-ci, une séquence d'ADN codant pour une protéine ayant l'activité biologique d'une 2-désoxyglucose-6-phosphate (2-DOG-6-P) phosphatase ; et
(c) fonctionnellement liées à celle-ci, des séquences régulatrices servant de signaux de polyadénylation et/ou de terminaison de transcription chez des plantes.

2. Molécule d'ADN recombinant de la revendication 1, dans laquelle la séquence d'ADN qui code pour une protéine ayant l'activité d'une 2-DOG-6-P phosphatase est choisie dans l'ensemble constitué par
(a) des séquences d'ADN comprenant une séquence nucléotidique qui code la séquence d'aminoacides indiquée dans SEQ ID n° 2 ;
(b) des séquences d'ADN comprenant la séquence nucléotidique indiquée dans SEQ ID n° 1 ;
(c) des séquences d'ADN comprenant une séquence nucléotidique qui s'hybride avec un brin complémentaire de la séquence nucléotidique de (a) ou (b) ;
(d) des séquences d'ADN comprenant une séquence nucléotidique qui est dégénérée par rapport à une séquence nucléotidique de (c), et
(e) des séquences d'ADN qui sont un dérivé, analogue ou fragment d'une séquence nucléotidique de (a), (b), (c) ou (d) et codant pour une protéine ayant une activité 2-DOG-6-P phosphatase.

3. Molécule d'ADN recombinant de la revendication 1 ou 2, dans laquelle la séquence d'ADN est issue d'une levure.

4. Molécule d'ADN recombinant de l'une quelconque des revendications 1 à 3, dans laquelle le promoteur est le promoteur 35S de CaMV.

5. Vecteur comprenant une molécule d'ADN recombinant de l'une quelconque des revendications 1 à 4.

6. Vecteur de la revendication 5, qui contient au moins une autre molécule d'ADN recombinant.

7. Vecteur de la revendication 6, dans lequel l'autre molécule d'ADN recombinant contient une séquence d'ADN qui code pour un peptide, une protéine, un ARN antisens, un ARN sens, un ARN viral ou un ribozyme.

8. Cellule hôte contenant une molécule d'ADN recombinant de l'une quelconque des revendications 1 à 4 ou un vecteur de l'une quelconque des revendications 5 à 7.

9. Nécessaire comprenant une molécule d'ADN recombinant de l'une quelconque des revendications 1 à 4 ou un vecteur de l'une quelconque des revendications 5 à 7, et éventuellement du 2-désoxyglucose ou un composé chimique fonctionnellement équivalent au 2-désoxyglucose.

10. Procédé pour la sélection de cellules végétales transformées, comprenant les étapes suivantes:
(a) obtention de cellules végétales ;
(b) introduction d'une molécule d'ADN recombinant de l'une quelconque des revendications 1 à 4 ou d'un vecteur de l'une quelconque des revendications 5 à 7 dans ces cellules végétales ; et
(c) sélection des cellules transformées avec succès sur des milieux contenant du 2-désoxyglucose ou sur des milieux contenant un composé chimique qui est fonctionnellement équivalent au 2-désoxyglucose.

11. Procédé de la revendication 10, dans lequel le vecteur de l'une quelconque des revendications 5 à 7 est transféré à des cellules végétales par l'intermédiaire d'*Agrobacterium tumefaciens*.

12. Procédé de la revendication 10, dans lequel la molécule d'ADN recombinant de l'une quelconque des revendications 1 à 4 ou le vecteur de l'une quelconque des revendications 5 à 7 est transféré à des cellules végétales par bombardement de particules.

13. Cellule végétale transgénique contenant une molécule d'ADN recombinant de l'une quelconque des revendications 1 à 4 ou un vecteur de l'une quelconque des revendications 5 à 7 ou produite selon le procédé de l'une quelconque des revendications 10 à 12.

14. Cellule végétale de la revendication 13, qui contient au moins un autre gène étranger.

15. Tissu végétal comprenant des cellules végétales de la revendication 13 ou 14 ou produit selon le procédé de l'une quelconque des revendications 10 à 12.

16. Plante transgénique contenant une cellule végétale de la revendication 13 ou 14 ou produite selon le procédé de l'une quelconque des revendications 10 à 12.

17. Produits de récolte de la plante de la revendication 16, comprenant des cellules végétales de la revendication 13 ou 14.

18. Matériel de propagation des plantes de la revendication 16, comprenant des cellules végétales de la revendication 13 ou 14.

19. Utilisation d'une molécule d'ADN qui comprend une séquence d'ADN telle que définie dans l'une quelconque des revendications 1 à 3, ou d'une molécule d'ADN recombinant de l'une quelconque des revendications 1 à 4 ou d'un vecteur de l'une quelconque des revendications 5 à 7, pour la production de plantes, cellules végétales et/ou tissu végétal transgéniques.

20. Utilisation d'une molécule d'ADN qui comprend une séquence d'ADN telle que définie dans l'une quelconque des revendications 1 à 3, ou d'une molécule d'ADN recombinant de l'une quelconque des revendications 1 à 4 ou d'un vecteur de l'une quelconque des revendications 5 à 7, en tant que marqueur sélectionnable dans la culture de cellules végétales et de tissus végétaux et/ou en phytogénétique.
